# EUROPÄISCHE PATENTSCHRIFT

(11) **EP 3 836 893 B1**
(45) Veröffentlichungstag und Bekanntmachung des Hinweises auf die Patenterteilung: **14.09.2022**
(21) Anmeldenummer: 19726374.2
(22) Anmeldetag: 22.05.2019
(51) Int. Cl.: A61K 8/58, A61Q 5/06, A61Q 5/10

(54) **VERFAHREN ZUM FÄRBEN VON KERATINISCHEM MATERIAL, UMFASSEND DIE ANWENDUNG VON EINER SILICIUMORGANISCHEN VERBINDUNG, EINEM OLIGOALKYLSILOXAN UND EINER FARBGEBENDEN VERBINDUNG**
METHOD FOR DYEING KERATINOUS MATERIAL, COMPRISING THE USE OF AN ORGANOSILICON COMPOUND, AN OLIGOALKYLSILOXANE AND A DYEING COMPOUND
PROCÉDÉ DESTINÉ À TEINDRE DE LA MATIÈRE KÉRATINIQUE, CONSISTANT EN L'APPLICATION D'UN COMPOSÉ ORGANIQUE AU SILICIUM, D'UN OLIGOALKYLSILOXANE ET D'UN COMPOSÉ COLORANT

(30) Priorität: 16.08.2018 DE 102018213813
(43) Veröffentlichungstag der Anmeldung: 23.06.2021
(73) Patentinhaber: Henkel AG & Co. KGaA, 40589 Düsseldorf (DE)
(72) Erfinder: LECHNER, Torsten, 40764 Langenfeld (DE); SCHOEPGENS, Juergen, 41366 Schwalmtal (DE); NOWOTTNY, Marc, 41069 Mönchengladbach (DE); WESER, Gabriele, 41472 Neuss (DE); SCHUMACHER, Ulrike, 40589 Düsseldorf (DE); KOLONKO, Claudia, 42857 Remscheid (DE); KRIENER, Caroline, 40229 Düsseldorf (DE); MATHIASZYK, Carsten, 45277 Essen (DE)
(86) Internationale Anmeldenummer: PCT/EP2019/063192
(87) Internationale Veröffentlichungsnummer: WO 2020/035186

(56) Entgegenhaltungen:
- WO-A1-2018/115059
- FR-A1- 2 936 414
- US-A- 5 411 585
- ARKLES B ET AL: "Factors contributing to the stability of alkoxysilanes in aqueous solution", 1. Januar 1992 (1992-01-01), SILANES AND OTHER COUPLING AGENTS, VSP, UTRECHT, NL, PAGE(S) 91 - 104, XP007918498, ISBN: 978-90-6764-142-5 Zusammenfassung

## Beschreibung

Gegenstand der vorliegenden Anmeldung ist ein Verfahren zum Färben von keratinischem Material, insbesondere menschlichen Haaren, welches die Anwendung von mindestens zwei verschiedenen Mitteln (a) und (b) umfasst. Das Mittel (a) enthält mindestens eine organische Siliciumverbindung und mindestens ein Oligoalkylsiloxan. Das Mittel (b) enthält mindestens eine farbgebende Verbindung aus der Gruppe der Pigmente und/oder der direktziehenden Farbstoffe.

Ein zweiter Gegenstand dieser Anmeldung ist eine Mehrkomponenten-Verpackungseinheit (Kit-of-parts) zum Färben von keratinischem Material, insbesondere menschlichen Haaren, welche getrennt voneinander konfektioniert in zwei verschiedenen Containern die Mittel (a) und (b) umfasst.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren.

EP 2168633 B1 und FR 2936414 A1 beschäftigten sich mit der Aufgabenstellung, langanhaltende Haarfärbungen unter Einsatz von Pigmenten zu erzeugen. Die Schriften lehrten, dass sich bei Verwendung einer Kombination aus Pigment, organischer Silicium-Verbindung, hydrophobem Polymer und einem Lösungsmittel auf Haaren Färbungen erzeugen lassen, die gegenüber Shampoonierungen besonders widerstandsfähig sein sollen. Als organischer Silicium-Verbindung wurde beispielsweise 3-Aminopropyltriethoxysilan eingesetzt.

In WO 2018/115059 A1 wird ein Färbeverfahren beschrieben, welches in mehreren Schritten abläuft. Ein Schritt umfasst die Anwendung eines Organosilans, und in einem weiteren Schritt wird ein direktziehender Farbstoffe auf die Haare appliziert. Auch mit diesem Verfahren sollen Färbungen mit guten Waschechtheiten erzielt werden. Die in dieser Schrift verwendeten Silane sind beispielsweise 3-Aminopropyltriethxoysilan und Methyltrimethoxysilan.

In den Färbeverfahren von EP 2168633 B1 und WO 2018/115059 A1 werden siliciumorganische Verbindungen aus der Gruppe der Silane eingesetzt, wobei die Molekülstruktur dieser Silane mindestens eine Hydroxygruppe und/oder hydrolysierbare Gruppe umfasst. Aufgrund der Anwesenheit der Hydroxygruppen bzw. hydrolysierbaren Gruppen handelt es sich bei den Silanen um reaktive Substanzen, die in Gegenwart von Wasser hydrolysieren bzw. oligomerisieren oder polymerisieren. Die durch Anwesenheit des Wassers initiierte Oligomerisierung bzw. Polymerisierung der Silane führt bei Anwendung auf dem Keratinmaterial letztendlich zur Ausbildung eines Films, welcher die farbgebenden Verbindungen fixiert und auf diese Weise sehr langlebige Färbungen erzeugt.

US 5411585 A beschreibt ein Verfahren zur Erhöhung der Stabilität wässriger Lösungen von Silanen, bei welchem quartäre Ammoniumverbindungen und Tenside eingesetzt werden. In dem Artikel "Factors contributing to the stability of alkoxysilanes in aqueous Solution" (B. Arkijes et. al, Silanes and Other Coupling Agents, S. 91 - 104, VSP 1992) wird auf den Einfluss der sterischen Hinderung von Silanen auf deren Stabilität in wässrigem Medium hingewiesen.

Bei näherer Untersuchung der in EP 2168633 B1 und WO 2018/115059 A1 offenbarten Färbeverfahren hat sich gezeigt, dass der Zeitpunkt der Filmbildung wesentlich für die Qualität des Farbergebnisses ist. Es wurde herausgefunden, dass optimale Färberesultate nur dann erhalten werden können, wenn die Ausbildung des Films genau auf den Zeitpunkt der Anwendung abgestimmt ist.

Findet die Oligomerisierung bzw. Polymerisierung der Silane zu früh oder zu schnell statt, bilden sich bereits große Konglomerate im Gebinde. In diesem Fall geliert die Formulierung, welche die Silane enthält, schon in dem Gefäß, in dem sie zur Verfügung gestellt wird. Die Erzeugung von intensiven oder lang anhaltenden Färbungen ist auf diesem Weg nicht mehr möglich.

Andererseits ist auch eine zu langsame Oligomerisierung bzw. Polymerisierung im Hinblick auf das Farbresultat von Nachteil, denn wenn sich der Film aus dem keratinischen Material nicht in ausreichender Schnelligkeit ausbilden kann, findet auch die Fixierung der farbgebenden Verbindungen auf dem Keratin nicht oder nur in unvollständigem Ausmaß statt. Folge der zu langsamen Polymerisierung sind daher zu schwache Farbintensitäten und ein viel zu schnelles Auswaschen der Färbung.

Eine große Herausforderung bei Färbeverfahren mit Silanen bleibt daher nach wie vor die Abstimmung der Polymerisierungsgeschwindigkeit auf den Färbeprozess. In erster Näherung besteht ein direkter Zusammenhang zwischen der Schnelligkeit der Polymerisierung und dem Wassergehalt des Färbemittels. Je höher der Wassergehalt ist, desto schneller polymerisieren die Silane.

Weitere Färbeversuchen haben jedoch gezeigt, dass die Empfindlichkeit der Silane gegenüber Wasser so hoch ist, dass auch durch Spuren von Luftfeuchtigkeit eine vorzeitige Hydrolyse in Gang gesetzt werden kann. Werden die Silane beispielsweise ohne Schutzgas umgefüllt, so ist bereits ein kurzzeitiger Kontakt mit Feuchtigkeit aus der Luft ausreichend, um eine viel zu früh einsetzende Hydrolyse zu initiieren. Die Lagerstabilität der in feuchter Luft produzierter Gebinde ist deshalb mangelhaft.

Es war die Aufgabe der vorliegenden Erfindung, ein Färbesystem bereitzustellen, das mit der oxidativen Färbung vergleichbare Echtheitseigenschaften besitzt. Insbesondere die Waschechtheiten sollten herausragend sein, hierbei sollte jedoch auf den Einsatz der sonst zu diesem Zweck üblicherweise eingesetzten Oxidationsfarbstoffvorprodukte verzichtet werden. Es wurde nach einer Technologie gesucht, die es ermöglicht, die aus dem Stand der Technik bekannten farbgebenden Verbindungen (wie beispielsweise Pigmente oder direktziehende Farbstoffe) in extrem dauerhafter Weise auf den Haaren zu fixieren. Die Produktion der Färbemittel sollte kostengünstig sein, und die Mittel selbst sollten eine sehr gute Lagerstabilität besitzen. Bei Anwendung der Mittel in einem Färbeverfahren sollten unabhängig von den bei der Produktion und der Anwendung herrschenden Bedingungen (wie z.B. Luftfeuchte und Temperatur) konstant gute Farbergebnisse erzielbar sein.

Überraschenderweise hat sich nun herausgestellt, dass die vorgenannte Aufgabe hervorragend gelöst werden kann, wenn keratinische Materialien, insbesondere Haare, mit einem Verfahren gefärbt werden, bei welchem mindestens zwei Mittel (a) und (b) auf die keratinischen Materialien (Haare) appliziert werden. Hierbei enthält das Mittel (a) mindestens eine organische Siliciumverbindung (aus der Gruppe der reaktiven Silane) und zusätzlich mindestens ein Oligoalkylsiloxan. Das Mittel (b) enthält mindestens eine farbgebende Verbindung aus der Gruppe der Pigmente und der direktziehenden Farbstoffe. Bei Einsatz der beiden Mittel (a) und (b) in einem Färbeverfahren konnten keratinische Fasern unabhängig von den bei Herstellung und Anwendung herrschenden Bedingungen in konstant hoher Farbintensität gefärbt werden. Zudem war die Waschechtheit der mit (a) und (b) gefärbten Haare hervorragend.

Ein erster Gegenstand der vorliegenden Erfindung ist ein Verfahren zum Färben von keratinischem Material, insbesondere menschlichen Haaren, umfassend die folgenden Schritte:
- Anwendung eines Mittels (a) auf dem keratinischem Material, wobei das Mittel (a) mindestens eine organische Siliciumverbindung enthält, die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst, und wobei das Mittel (a) weiterhin mindestens ein Oligoalkylsiloxan enthält, und
- Anwendung eines Mittels (b) auf dem keratinischen Material, wobei das Mittel (b) mindestens eine farbgebende Verbindung aus der Gruppe der Pigmente und/oder der direktziehenden Farbstoffe enthält.

### keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

### Mittel (a) und (b)

Im Rahmen des erfindungsgemäßen Verfahrens werden die Mittel (a) und (b) auf dem keratinischen Material, insbesondere den menschlichen Haaren, appliziert. Die beiden Mittel (a) und (b) sind voneinander verschieden.

Mit anderen Worten ist ein erster Gegenstand der vorliegenden Erfindung ein Verfahren zum Färben von keratinischem Material, insbesondere menschlichen Haaren, umfassend die folgenden Schritte:
- Anwendung eines Mittels (a) auf dem keratinischem Material, wobei das Mittel (a) mindestens eine organische Siliciumverbindung enthält, die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst, und wobei das Mittel (a) weiterhin mindestens ein Oligoalkylsiloxan enthält, und
- Anwendung eines Mittels (b) auf dem keratinischen Material, wobei das Mittel (b) mindestens eine farbgebende Verbindung aus der Gruppe der Pigmente und/oder der direktziehenden Farbstoffe enthält, wobei die beiden Mittel (a) und (b) voneinander verschieden sind.

### Mittel (a)

Als ersten erfindungwesentlichen Inhaltsstoff enthält das Mittel (a) mindestens eine organische Siliciumverbindung, die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst.

Wie bereits beschrieben sind die im Mittel (a) enthaltenen organischen Siliciumverbindungen bzw. organischen Silane reaktive Verbindungen.

Das Mittel (a) enthält die organischen Siliciumverbindung(en), insbesondere das oder die organischen Silane in einem kosmetischen Träger, der wasserhaltig, wasserarm oder auch wasserfrei sein kann. Zudem kann der kosmetische Träger flüssig, gelartig, cremeförmig, pulverförmig oder auch fest (z.B. in Form einer Tablette oder eines Presslings) sein. Bevorzugt ist der kosmetische Träger des Mittels (a) ein wässriger oder wässrig-alkoholischer Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind.

Der kosmetische Träger ist bevorzugt wasserhaltig, was bedeutet, dass der Träger - bezogen auf sein Gewicht - mindestens 2 Gew.-% Wasser enthält. Bevorzugt liegt der Wassergehalt oberhalb von 5 Gew.-%, weiter bevorzugt oberhalb von 10 Gew.-% noch weiter bevorzugt oberhalb von 15 Gew.-%. Der kosmetische Träger kann auch wässrig-alkoholisch sein. Unter wässrig-alkoholischen Lösungen sind im Sinne der vorliegenden Erfindung wässrige Lösungen enthaltend 2 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Der Begriff "Mittel zur Färbung" wird im Rahmen dieser Erfindung für eine durch Einsatz von Pigmenten und/oder direktziehenden Farbstoffen hervorgerufene Farbgebung des Keratinmaterials, insbesondere des Haares, verwendet. Bei dieser Färbung lagern sich die vorgenannten farbgebenden Verbindungen in einem besonders homogenen und glatten Film an der Oberfläche des Keratinmaterials ab oder diffundieren in die Keratinfaser hinein. Der Film bildet sich in situ durch Oligomerisierung bzw. Polymerisierung des oder der organischen Siliciumverbindungen, sowie durch die Wechselwirkung von organischer Silicumverbindung mit der farbgebender Verbindung.

### Organische Siliciumverbindungen

Als ersten erfindungswesentlichen Bestandteil enthält das Mittel (a) mindestens eine organische Siliciumverbindung. Erfindungsgemäße organische Siliciumverbindungen werden ausgewählt aus Silanen mit einem, zwei oder drei Siliciumatomen.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die erfindungsgemäßen organische Siliciumverbindungen sind Verbindungen, die eine bis drei Silicumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Das Mittel (a) enthält mindestens eine organischen Siliciumverbindung, die aus Silanen mit einem, zwei oder drei Siliciumatomen ausgewählt ist, wobei die organische Siliciumverbindung eine oder mehrere Hydroxylgruppen oder hydrolysierbare Gruppen pro Molekül umfasst.

Im Rahmen einer ganz besonders bevorzugten Ausführungsform ist ein erfindungsgemäßes Verfahren gekennzeichnet durch die Anwendung eines Mittels (a) auf dem keratinischem Material, wobei das Mittel (a) mindestens eine organische Siliciumverbindung enthält, die aus Silanen mit einem, zwei oder drei Siliciumatomen ausgewählt ist, wobei die organische Siliciumverbindung außerdem eine oder mehrere basische chemische Funktionen und eine oder mehrere Hydroxylgruppen oder hydrolysierbare Gruppen pro Molekül umfasst.

Bei dieser basischen Gruppe kann es sich beispielsweise um eine Aminogruppe, eine Alkylaminogruppe oder um eine Dialkylaminogruppe handeln, die bevorzugt über einen Linker mit einem Siliciumatom verbunden ist. Bevorzugt handelt es sich bei der basischen Gruppe um eine Aminogruppe, eine C₁-C₆-Alkylaminogruppe oder um eine Di(C₁-C₆)alkylaminogruppe.

Bei der oder den hydrolysierbaren Gruppen handelt es sich bevorzugt um eine C₁-C₆-Alkoxygruppe, insbesondere um eine Ethoxygruppe oder um eine Methoxygruppe. Es ist bevorzugt, wenn die hydrolysierbare Gruppe direkt an das Siliciumatom gebunden vorliegt. Handelt es sich beispielsweise bei der hydrolysierbaren Gruppe um eine Ethoxygruppe, so enthält die organische Siliciumverbindung bevorzugt eine Struktureinheit R'R"R‴Si-O-CH2-CH3. Die Reste R', R" und R‴ stellen hierbei die drei übrigen freien Valenzen des Siliciumatoms dar.

Ein ganz besonders bevorzugtes erfindungsgemäßes Verfahren ist dadurch gekennzeichnet, dass das Mittel (a) mindestens eine organische Siliciumverbindung enthält, die aus Silanen mit einem, zwei oder drei Siliciumatomen ausgewählt ist, wobei die organische Siliciumverbindung bevorzugt eine oder mehrere basische chemische Funktionen und eine oder mehrere Hydroxylgruppen oder hydrolysierbare Gruppen pro Molekül umfasst.

Ganz besonders gute Ergebnisse konnten erhalten werden, wenn das erfindungsgemäße Mittel (a) mindestens eine organische Siliciumverbindung der Formel (I) und/oder (II) enthält.

Die Verbindungen der Formeln (I) und (II) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

In einer weiteren ganz besonders bevorzugten Ausführungsform ist ein erfindungsgemäßes Verfahren dadurch gekennzeichnet, dass auf dem keratinischen Material (bzw. den menschlichen Haaren) ein Mittel (a) angewendet wird, wobei das Mittel (a) mindestens eine organische Siliciumverbindung (a) der Formel (I) und/oder (II) enthält,

R₁R₂N-L-Si(OR₃)ₐ(R₄)_{b} (I),

wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃ für ein Wasserstoffatom oder eine C₁-C₆-Alkylgruppe steht,
- R₄ für eine C₁-C₆-Alkylgruppe steht
- a, für eine ganze Zahl von 1 bis 3 steht, und
- b für die ganze Zahl 3 - a steht,

   (R₅O)_{c}(R₆)_{d}Si-(A)ₑ-[NR₇-(A')]_{f}-[O-(A")]_{g}-[NR₈-(A'")]ₕ-Si(R₆')_{d'}(OR₅')_{c'} (II),

   wobei
- R5, R5', R5" unabhängig voneinander für ein Wasserstoffatom oder für eine C₁-C₆-Alkylgruppe stehen,
- R6, R6' und R6" unabhängig voneinander für eine C₁-C₆-Alkylgruppe stehen,
- A, A', A", A'" und A"" unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (III) stehen
- (A"")-Si(R6")d"(OR5")c" (III),
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
- mit der Maßgabe, dass mindestens einer der Reste aus e, f, g und h von 0 verschieden ist.

Die Substituenten R₁, R₂, R₃, R₄, R₅, R₅', R₅", R₆, R₆', R₆", R₇, R₈, L, A, A', A", A‴ und A"" in den Verbindungen der Formel (I) und (II) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In den organischen Siliciumverbindungen der Formel (I)

R₁R₂N-L-Si(OR₃)ₐ(R₄)_{b} (I),

stehen die Reste R₁ und R₂ unabhängig voneinander für ein Wasserstoffatom oder eine C₁-C₆-Alkylgruppe. Ganz besonders bevorzugt stehen die Reste R₁ und R₂ beide für ein Wasserstoffatom.

Im Mittelteil der organischen Siliciumverbindung befindet sich die Struktureinheit oder der Linker -L-der für eine lineare oder verzweigte, zweiwertige C₁-C₂₀-Alkylengruppe steht.

Bevorzugt steht -L- für eine lineare, zweiwertige C₁-C₂₀-Alkylengruppe. Weiter bevorzugt steht -L- für eine lineare zweiwertige C₁-C₆-Alkylengruppe. Besonders bevorzugt steht -L- für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die erfindungsgemäßen organischen Siliciumverbindungen der Formel (I)

R₁R₂N-L-Si(OR₃)ₐ(R₄)_{b} (I),

tragen jeweils ein einem Ende die Silicium-haltige Gruppierung -Si(OR₃)ₐ(R₄)_{b}.

In der endständigen Struktureinheit -Si(OR₃)ₐ(R₄)_{b} steht der Rest R₃ für ein Wasserstoffatom oder eine C₁-C₆-Alkylgruppe, und der Rest R₄ steht für eine C₁-C₆-Alkylgruppe. Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Färbungen mit den besten Waschechtheiten konnten erhalten werden, wenn das erfindungsgemäße Mittel mindestens eine organische Siliciumverbindung der Formel (I) enthält, in welcher die Reste R₃, R₄ unabhängig voneinander für eine Methylgruppe oder für eine Ethylgruppe stehen.

Weiterhin konnten Färbungen mit den besten Waschechtheiten erhalten werden, wenn das erfindungsgemäße Mittel mindestens eine organische Siliciumverbindung der Formel (I) enthält, in welcher der Rest a für die Zahl 3 steht. In diesem Fall steht der Rest b für die Zahl 0.

In einer weiteren bevorzugten Ausführungsform ist ein erfindungsgemäßes Mittel dadurch gekennzeichnet, dass es (a) mindestens eine organische Siliciumverbindung der Formel (I) enthält,
wobei
- R₃, R₄ unabhängig voneinander für eine Methylgruppe oder für eine Ethylgruppe stehen und
- a für die Zahl 3 steht und
- b für die Zahl 0 steht.

In einer weiteren bevorzugten Ausführungsform ist ein erfindungsgemäßes Verfahren dadurch gekennzeichnet, dass das Mittel (a) mindestens eine organische Siliciumverbindung der Formel (I) enthält,

R₁R₂N-L-Si(OR₃)ₐ(R₄)_{b} (I),

wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃ für ein Wasserstoffatom, eine Ethylgruppe oder eine Methylgruppe steht,
- R₄ für eine Methylgruppe oder für eine Ethylgruppe steht,
- a für die Zahl 3 steht und
- b für die Zahl 0 steht.

Zur Lösung der erfindungsgemäßen Aufgabenstellung besonders gut geeignete organische Silicumverbindungen der Formel (I) sind
- (3-Aminopropyl)triethoxysilan 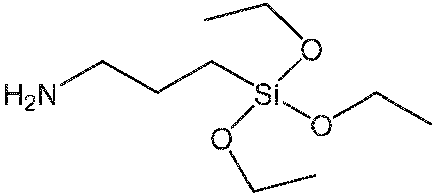
- (3-Aminopropyl)trimethoxysilan 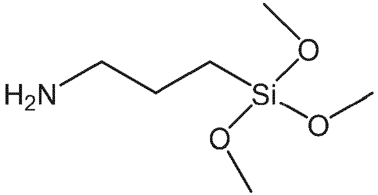
- 1-(3-Aminopropyl)silantriol 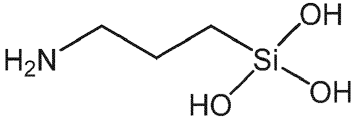
- (2-Aminoethyl)triethoxysilan 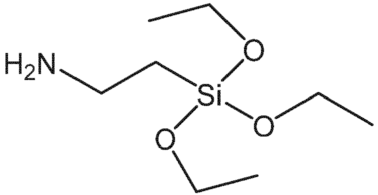
- (2-Aminoethyl)trimethoxysilan 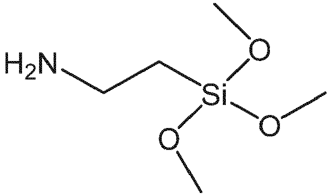
- 1-(2-Aminoethyl)silantriol 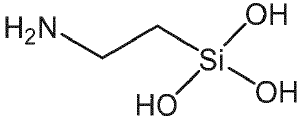
- (3-Dimethylaminopropyl)triethoxysilan 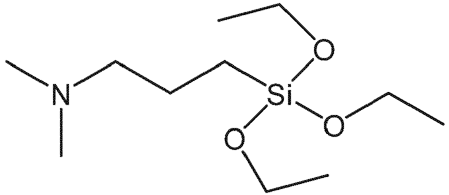
- (3-Dimethylaminopropyl)trimethoxysilan 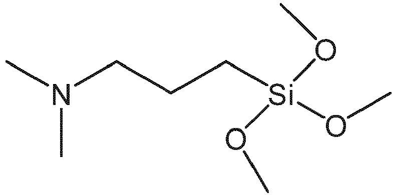
- 1-(3-Dimethylaminopropyl)silantriol 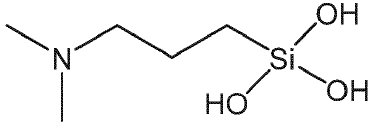
- (2-Dimethylaminoethyl)triethoxysilan. 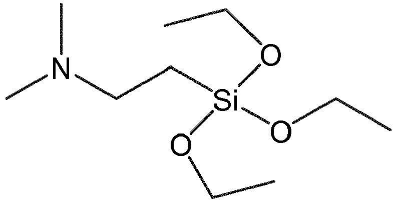
- (2-Dimethylaminoethyl)trimethoxysilan und/oder 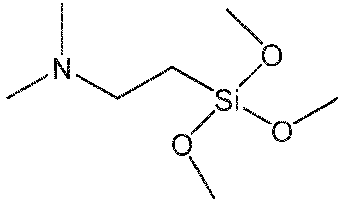
- 1-(2-Dimethylaminoethyl)silantriol

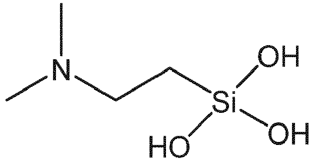

In einer weiteren bevorzugten Ausführungsform ist ein erfindungsgemäßes Verfahren dadurch gekennzeichnet, dass das Mittel (a) mindestens eine organische Siliciumverbindung der Formel (I) enthält, die ausgewählt ist aus der Gruppe aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- 1-(3-Dimethylaminopropyl)silantriol
- (2-Dimethylaminoethyl)triethoxysilan.
- (2-Dimethylaminoethyl)trimethoxysilan und/oder
- 1-(2-Dimethylaminoethyl)silantriol.

Die vorgenannten organische Siliciumverbindung der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Im Rahmen einer weiteren Ausführungsform enthält das erfindungsgemäße Mittel mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A'")]ₕ-Si(R₆')_{d'}(OR₅')_{c'} (II).

Die erfindungsgemäßen siliciumorganischen Verbindungen der Formel (II) tragen jeweils an ihren beiden Enden die Silicium-haltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}- und -[O-(A")]_{g}- und -[NR₈-(A'")]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine erfindungsgemäße organischen Siliciumverbindung der Formel (II) mindestens eine Gruppierung aus der Gruppe aus -(A)- und - [NR₇-(A')]- und -[O-(A")]- und -[NR₈-(A'")]-.

In den beiden endständigen Struktureinheiten (R₅O)_{c}(R₆)_{d}Si- und - Si(R₆')_{d'}(OR₅')_{c'},stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' fürdie Zahl 3 steht, dann ist d' gleich 0. Wenn c' für die Zahl 2 steht, dann ist d' gleich 1. Wenn c' für die Zahl 1 steht, dann ist d' gleich 2.

Färbungen mit den besten Waschechtheiten konnten erhalten werden, wenn die Reste c und c' beide für die Zahl 3 stehen. In diesem Fall stehen d und d' beide für die Zahl 0.

In einer weiteren bevorzugten Ausführungsform ist ein erfindungsgemäßes Verfahren dadurch gekennzeichnet, dass das Mittel (a) mindestens eine organische Siliciumverbindung der Formel (II) enthält,

(R₅O)_{c}(R₆)_{d}Si-(A)ₑ-[NR₇-(A')]_{f}-[O-(A")]_{g}-[NR₈-(A'")]ₕ-Si(R₆')_{d'}(OR₅')_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die erfindungsgemäßen organischen Siliciumverbindung der Formel (IIa)

(R₅O)₃Si-(A)e-[NR₇-(A')]_{f}-[O-(A")]g-[NR₈-(A'")]ₕ-Si(OR₅')₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A'")]ₕ-sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der Waschechtheit erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die erfindungsgemäßen organischen Siliciumverbindung der Formel (IIb)

(R₅O)_{c}(R₆)_{d}Si-(A)-[NR₇-(A')]-Si(R₆')_{d'}(OR₅')_{c'} (IIb).

Die Reste A, A', A", A'" und A"" stehen unabhängig voneinander für eine lineare oder verzweigte, zweiwertige C₁-C₂₀-Alkylengruppe. Bevorzugt stehen die Reste A, A', A", A‴ und A"" unabhängig voneinander für eine lineare, zweiwertige C₁-C₂₀-Alkylengruppe. Weiter bevorzugt stehen die Reste A, A', A", A‴ und A"" unabhängig voneinander für eine lineare zweiwertige C₁-C₆-Alkylengruppe. Besonders bevorzugt stehen die Reste A, A', A", A‴ und A"" unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und A"" für eine Propylengruppe (-CH₂-CH₂-CH₂-).

Wenn der Rest f für die Zahl 1 steht, dann enthält die erfindungsgemäße organische Siliciumverbindung der Formel (II) eine strukturelle Gruppierung -[NR7-(A')]-.

Wenn der Rest h für die Zahl 1 steht, dann enthält die erfindungsgemäße organische Siliciumverbindung der Formel (II) eine strukturelle Gruppierung -[NR₈-(A'")]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkylgruppe oder eine Gruppierung der Formel (III)

- (A"")-Si(R6")d"(OR5")c" (III).

Ganz besonders bevorzugt stehen die Reste R7 und R8 unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die erfindungsgemäße organische Silicumverbindung die Gruppierung [NR₇-(A')], aber nicht die Gruppierung -[NR₈-(A'")]. Steht nun der Rest R7 für eine Gruppierung der Formel (III), so enthält das VorbehandlungsMittel (a) eine organische Silicumverbindung mit 3 reaktiven Silan-Gruppen.

In einer weiteren bevorzugten Ausführungsform ist ein erfindungsgemäßes Verfahren dadurch gekennzeichnet, dass das Mittel (a) mindestens eine organische Siliciumverbindung der Formel (II) enthält,

(R₅O)_{c}(R₆)_{d}Si-(A)ₑ-[NR₇-(A')]_{f}-[O-(A")]_{g}-[NR₈-(A'")]ₕ-Si(R₆')_{d'}(OR₅')_{c'} (II),

wobei
- e und f beide für die Zahl 1 stehen,
- g und h beide für die Zahl 0 stehen,
- A und A' unabhängig voneinander für eine lineare, zweiwertige C₁-C₆-Alkylengruppe stehen und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform ist ein erfindungsgemäßes Verfahren dadurch gekennzeichnet, dass das Mittel (a) mindestens eine organische Siliciumverbindung der Formel (II) enthält, wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂-) oder eine Propylengruppe (-CH₂-CH₂-CH₂) stehen,
und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der erfindungsgemäßen Aufgabenstellung gut geeignete organische Silicumverbindungen der Formel (II) sind
- 3-(Trimethoxysilyl)-N-[3-(trimethoxysilyl)propyl]-1-propanamin 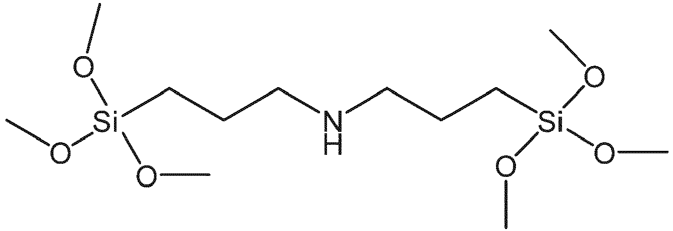
- 3-(Triethoxysilyl)-N-[3-(triethoxysilyl)propyl]-1-propanamin 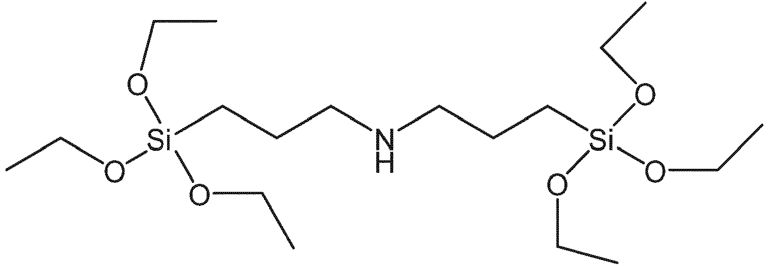
- N-Methyl-3-(trimethoxysilyl)-N-[3-(trimethoxysilyl)propyl]-1-propanamin 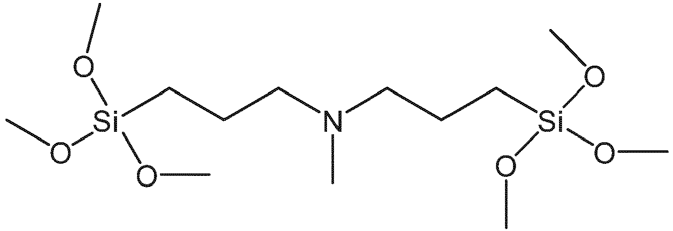
- N-Methyl-3-(triethoxysilyl)-N-[3-(triethoxysilyl)propyl]-1-propanamin 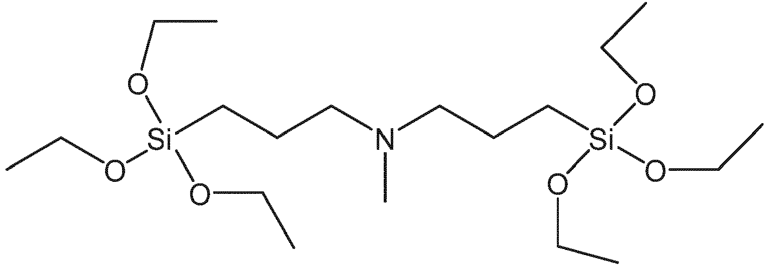
- 2-[Bis[3-(trimethoxysilyl)propyl]amino]-ethanol 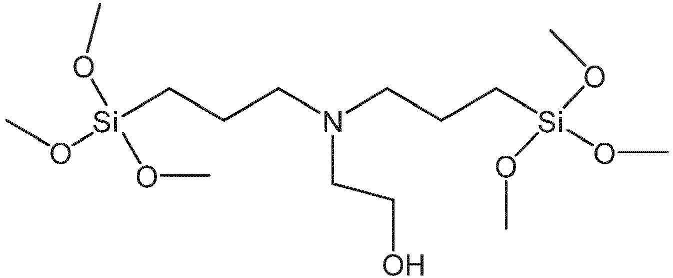
- 2-[Bis[3-(triethoxysilyl)propyl]amino]-ethanol 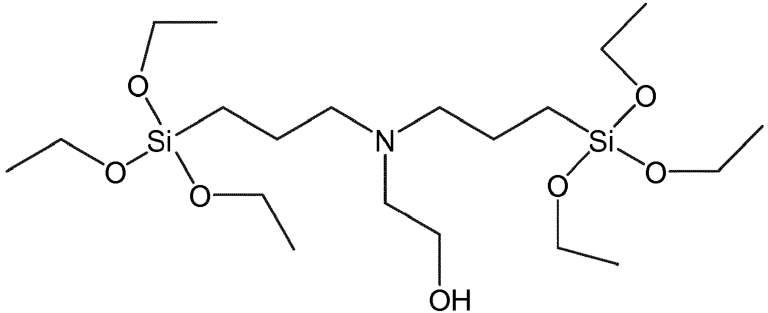
- 3-(Trimethoxysilyl)-N,N-bis[3-(trimethoxysilyl)propyl]-1-propanamin 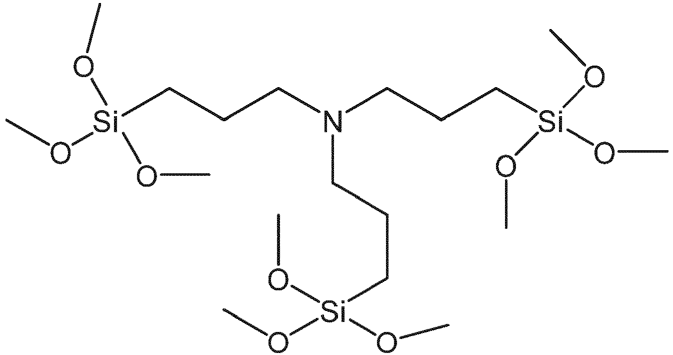
- 3-(Triethoxysilyl)-N,N-bis[3-(triethoxysilyl)propyl]-1-propanamin 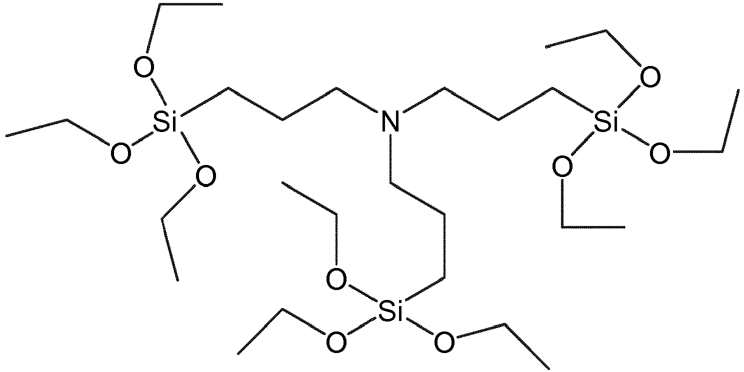
- N1,N1-Bis[3-(trimethoxysilyl)propyl]-1,2-ethanediamin, 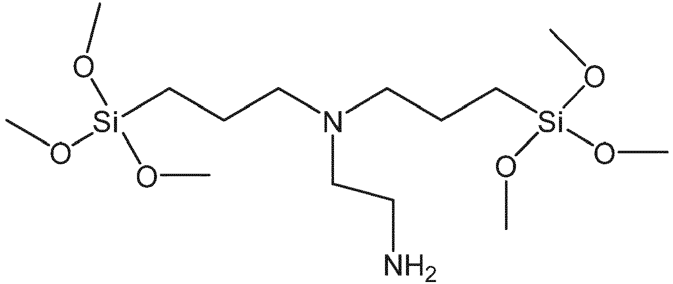
- N1,N1-Bis[3-(triethoxysilyl)propyl]-1,2-ethanediamin, 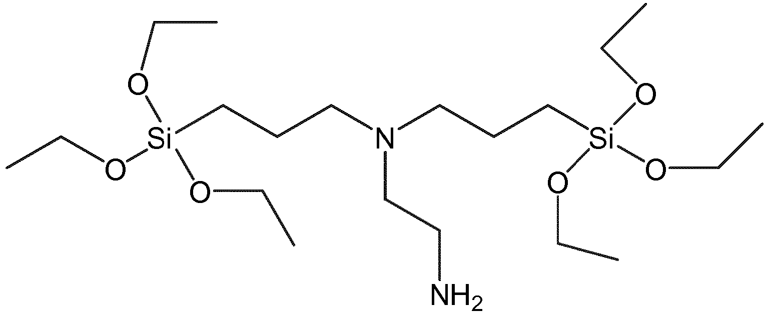
- N,N-Bis[3-(trimethoxysilyl)propyl]-2-propen-1-amin 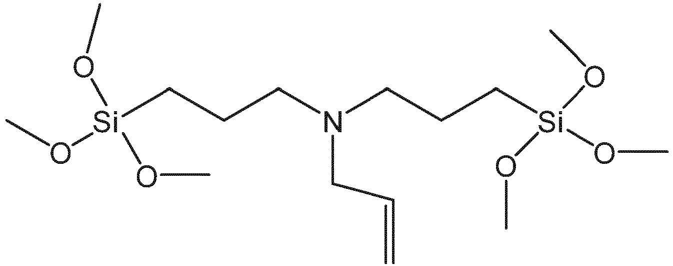
- N,N-Bis[3-(triethoxysilyl)propyl]-2-propen-1-amin

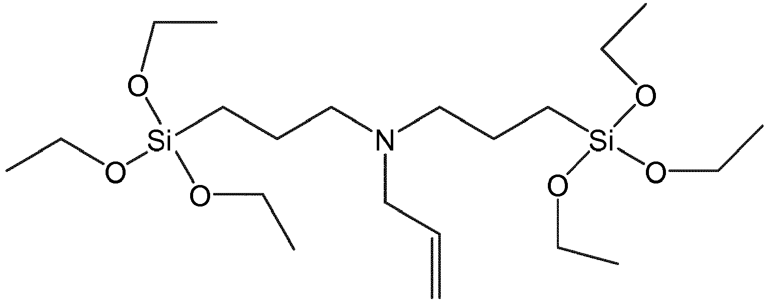

Die vorgenannten organische Siliciumverbindung der Formel (II) sind kommerziell erhältlich. Bis(trimethoxysilylpropyl)amine mit der CAS-Nummer 82985-35-1 kann beispielsweise von Sigma-Aldrich käuflich erworben werden.

Bis[3-(triethoxysilyl)propyl]amine mit der CAS-Nummer 13497-18-2 kann zum Beispiel von Sigma-Aldrich käuflich erworben werden.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

In einer weiteren bevorzugten Ausführungsform ist ein erfindungsgemäßes Mittel gekennzeichnet, dass es (a) mindestens eine organische Siliciumverbindung der Formel (II) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und/oder
- N,N-Bis[3-(triethoxysilyl)propyl]-2-Propen-1-amin.

In weiteren Färbeversuchen hat es sich ebenfalls als ganz besonders vorteilhaft herausgestellt, wenn das im erfindungsgemäßen Verfahren auf dem keratinischen Material angewendete Mittel (a) mindestens eine organische Siliciumverbindung der Formel (IV) enthält

R₉Si(OR₁₀)ₖ(R₁₁)ₘ (IV).

Die Verbindungen der Formel (IV) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Das bzw. die organischen Siliciumverbindungen der Formel (IV) können auch als Silane vom Typ der Alkyl-alkoxy-silane oder der Alkyl-hydroxy-silane bezeichnet werden,

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist ein erfindungsgemäßes Verfahren dadurch gekennzeichnet, dass das Mittel (a) mindestens eine organische Siliciumverbindung der Formel (IV) enthält.

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist ein erfindungsgemäßes Verfahren dadurch gekennzeichnet, dass das Mittel (a) zusätzlich zu der oder den organischen Siliicumverbindungen der Formel (I) mindestens eine weitere organische Siliciumverbindung der Formel (IV) enthält

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist ein erfindungsgemäßes Verfahren dadurch gekennzeichnet, dass das Mittel (a) zusätzlich zu der oder den organischen Siliicumverbindungen der Formel (II) mindestens eine weitere organische Siliciumverbindung der Formel (IV) enthält

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist ein erfindungsgemäßes Verfahren dadurch gekennzeichnet, dass das Mittel (a) zusätzlich zu der oder den organischen Siliicumverbindungen der Formel (I) und/oder (II) mindestens eine weitere organische Siliciumverbindung der Formel (IV) enthält

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In den organischen Siliciumverbindungen der Formel (IV) steht der Rest R₉ für eine C₁-C₁₂-Alkylgruppe. Diese C₁-C₁₂-Alkylgruppe ist gesättigt und kann linear oder verzweigt sein. Bevorzugt steht R9 für eine lineare C₁-C₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht R₉ für eine Methylgruppe, eine Ethylgrurppe oder eine n-Octylgruppe.

In den organischen Siliciumverbindungen der Forml (IV) steht der Rest R₁₀ für ein Wasserstoffatom oder eine C₁-C₆-Alkylgruppe. Besonders bevorzugt steht R10 für eine Methylgruppe oder für eine Ethylgruppe.

In den organischen Siliciumverbindungen der Forml (IV) steht der Rest R₁₁ für eine C₁-C₆-Alkylgruppe. Besonders bevorzugt steht R11 für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Färbungen mit den besten Waschechtheiten konnten erhalten werden, wenn im Verfahren ein Mittel (a) eingesetzt wurde, welches mindestens eine organische Siliciumverbindung der Formel (IV) enthält, in welcher der Rest k für die Zahl 3 steht. In diesem Fall steht der Rest m für die Zahl 0.

Zur Lösung der erfindungsgemäßen Aufgabenstellung besonders gut geeignete organische Silicumverbindungen der Formel (IV) sind
- Methyltrimethoxysilan 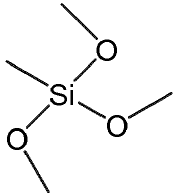
- Methyltriethoxysilan 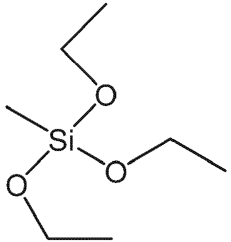
- Ethyltrimethoxysilan 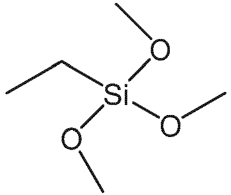
- Ethyltriethoxysilan 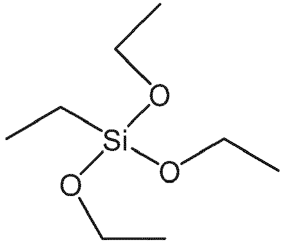
- n-Hexyltrimethoxysilan 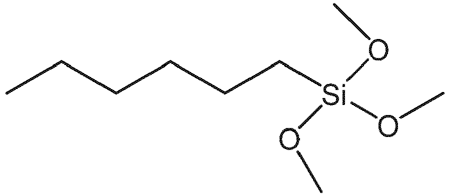
- n-Hexyltriethoxysilan 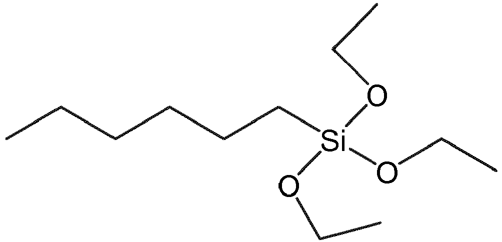
- n-Octyltrimethoxysilan 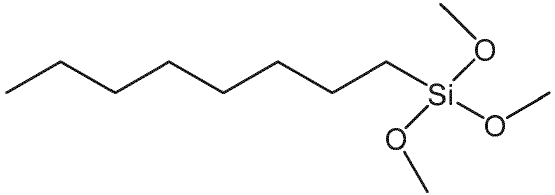
- n-Octyltriethoxysilan 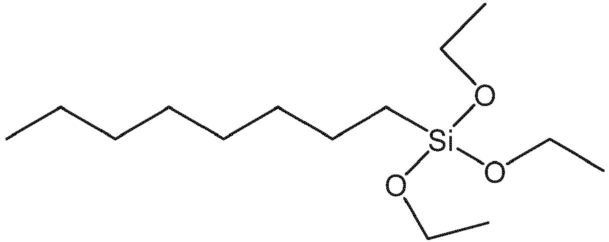
- n-Dodecyltrimethoxysilan und/oder 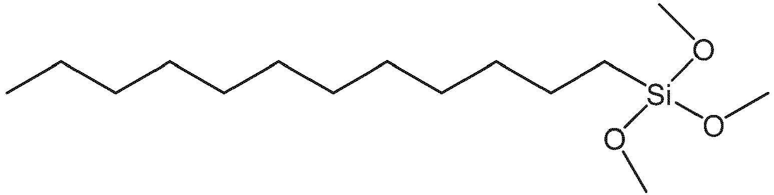
- n-Dodecyltriethoxysilan.

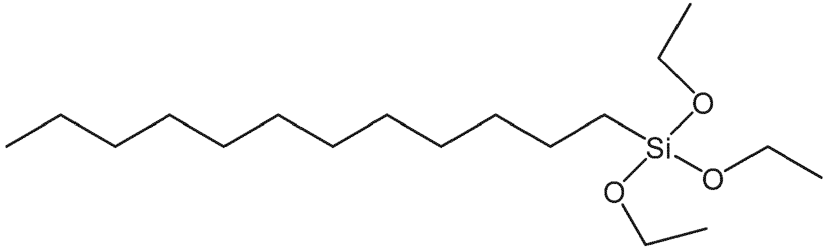

In einer weiteren bevorzugten Ausführungsform ist ein erfindungsgemäßes Verfahren dadurch gekennzeichnet, dass das Mittel (a) mindestens eine organische Siliciumverbindung der Formel (IV) enthält, die ausgewählt ist aus der Gruppe aus
- Methyltrimethoxysilan
- Methyltriethoxysilan
- Ethyltrimethoxysilan
- Ethyltriethoxysilan
- Hexyltrimethoxysilan
- Hexyltriethoxysilan
- Octyltrimethoxysilan
- Octyltriethoxysilan
- Dodecyltrimethoxysilan und/oder
- Dodecyltriethoxysilan.

In einer explizit ganz besonders bevorzugten Ausführungsform ist ein erfindungsgemäßes Verfahren dadurch gekennzeichnet, dass auf dem keratinischen Material ein Mittel (a) angewendet wird, welches mindestens eine organische Silicumverbindungen der Formel (I) enthält, welche aus der Gruppe aus (3-Aminopropyl)triethoxysilan und (3-Aminopropyl)trimethoxysilan ausgewäht ist, und zusätzlcih mindestens eine organische Silicumverbindungen der Formel (IV) enthält, welche aus der Gruppe aus Methyltrimethoxysilan, Methyltriethoxysilan, Ethyltrimethoxysilan und Ethyltriethoxysilan ausgewhält ist.

Bei den zuvor beschriebenen organischen Siliciumverbindungen handelt es sich um reaktive Verbindungen. In diesem Zusammenhang hat es sich als bevorzugt herausgestellt, wenn das erfindungsgemäße Mittel (a) - bezogen auf das Gesamtgewicht des Mittels (a) - eine oder mehrere organische Siliciumverbindungen in einer Gesamtmenge von 0,1 bis 20,0 Gew.-%, bevorzugt 1,0 bis 15,0 Gew.-% und besonders bevorzugt 2,0 bis 8,0 Gew.-% enthält.

In einer weiteren bevorzugten Ausführungsform ist ein erfindungsgemäßes Verfahren dadurch gekennzeichnet, dass das Mittel (a) - bezogen auf das Gesamtgewicht des Mittels (a) - eine oder mehrere organische Siliciumverbindungen in einer Gesamtmenge von 0,1 bis 20,0 Gew.-%, bevorzugt 1,0 bis 15,0 Gew.-% und besonders bevorzugt 2,0 bis 8,0 Gew.-% enthält.

Zur Erzielung von besonders guten Färbeergebnissen ist es insbesondere von Vorteil, die organische Siliciumverbindungen der Formel (I) und/oder (II) in bestimmten Mengenbereichen im Mittel (a) einzusetzen. Besonders bevorzugt enthält das Mittel (a) - bezogen auf das Gesamtgewicht des Mittels (a) - ein oder mehrere organische Siliciumverbindungen der Formel (I) und/oder (II) in einer Gesamtmenge von 0,1 bis 10,0 Gew.-%, bevorzugt 0,5 bis 5,0 Gew.-% und besonders bevorzugt 1,0 bis 3,0 Gew.-%.

In einer weiteren bevorzugten Ausführungsform ist ein erfindungsgemäßes Verfahren dadurch gekennzeichnet, dass das Mittel (a) - bezogen auf das Gesamtgewicht des Mittels (a) - eine oder mehrere organische Siliciumverbindungen der Formel (I) und/oder (II) in einer Gesamtmenge von 0,1 bis 10,0 Gew.-%, bevorzugt 0,5 bis 5,0 Gew.-% und besonders bevorzugt 1,0 bis 3,0 Gew.-% enthält.

Weiterhin hat es sich als ganz besonders bevorzugt herausgestellt, wenn auch das oder die organische Siliciumverbindungen der Formel (IV) in bestimmten Mengenbereichen im Mittel (a) enthalten sind. Besonders bevorzugt enthält das Mittel (a) - bezogen auf das Gesamtgewicht des Mittels (a) - eine oder mehrere organische Siliciumverbindungen der Formel (IV) in einer Gesamtmenge von 0,1 bis 20,0 Gew.-%, bevorzugt 2,0 bis 15,0 Gew.-% und besonders bevorzugt 4,0 bis 9,0 Gew.-%.

In einer weiteren bevorzugten Ausführungsform ist ein erfindungsgemäßes Verfahren dadurch gekennzeichnet, dass das Mittel (a) - bezogen auf das Gesamtgewicht des Mittels (a) - eine oder mehrere organische Siliciumverbindungen der Formel (IV) in einer Gesamtmenge von 0,1 bis 20,0 Gew.-%, bevorzugt 2,0 bis 15,0 Gew.-% und besonders bevorzugt 4,0 bis 9,0 Gew.-% enthält.

### Oligoalkylsiloxane

Als zweiten erfindungswesentlichen Bestandteil enthält das Mittel (a) mindestens ein Oligoalkylsiloxan.

Unter Oligoalkylsiloxanen werden im Sinne der Erfindung oligomere Siloxane verstanden, die linear oder cyclisch sein können.

Bevorzugte lineare Oligoalkylsiloxane sind Verbindungen der allgemeinen Formel (V) 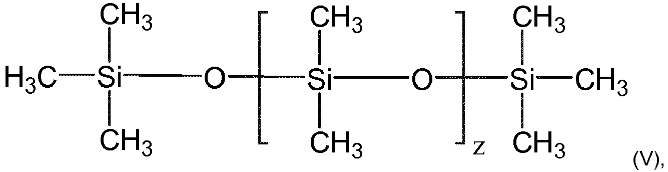 wobei z für eine ganze Zahl von 0 bis 10 steht. Bevorzugt steht z für die Zahlen 0, 1, 2 oder 3.

Ganz besonders bevorzugte lineare Oligoalkylsiloxane sind beispielsweise
- Hexamethyldisiloxan 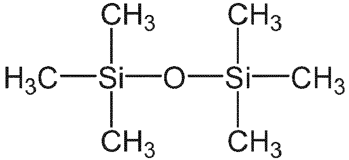
- Octamethyltrisiloxan 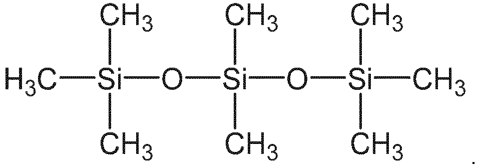
- Decamethyltetrasiloxan

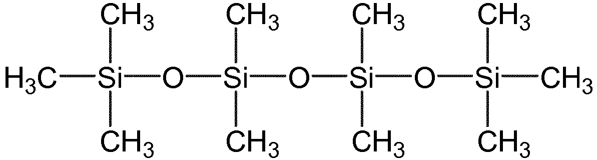

Hexamethyldisiloxan besitzt die CAS-Nummer 107-46-0 und kann beispielsweise bei Sigma-Aldrich kommerziell erworben werden.

Octamethyltrisiloxan besitzt die CAS-Nummer 107-51-7 und ist ebenfalls bei Sigma-Aldrich kommerziell erhältlich.

Decamethyltetrasiloxan trägt die CAS-Nummer 141-62-8 und ist ebenfalls bei Sigma-Aldrich kommerziell erhältlich.

Bevorzugte cyclische Oligoalkylsiloxane sind Verbindungen der allgemeinen Formel (VI) 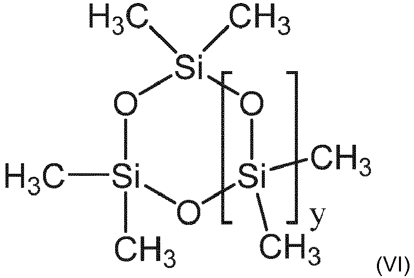 wobei y für eine ganze Zahl von 1 bis 5 steht. Bevorzugt steht z für die Zahlen 1, 2 oder 3.

Ganz besonders bevorzugte cyclische Oligoalkylsiloxane sind beispielsweise
- Hexamethylcyclotrisiloxan
- Octamethylcyclotetrasiloxan
- Decamethylcyclopentasiloxan

In einer weiteren bevorzugten Ausführungsform ist ein erfindungsgemäßes Verfahren gekennzeichnet,
dass das Mittel (a) mindestens ein lineares und/oder cyclisches Oligoalkylsiloxan enthält.

In einer weiteren bevorzugten Ausführungsform ist ein erfindungsgemäßes Verfahren gekennzeichnet,
dass das Mittel (a) mindestens ein Oligoalkylsiloxan der Formel (V) und/oder (VI) enthält, 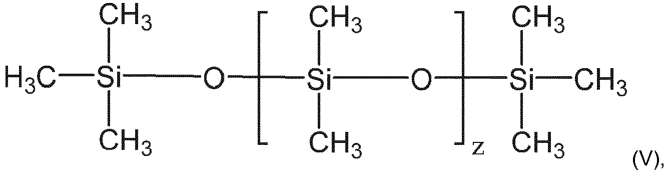 wobei z für eine ganze Zahl von 0 bis 10, bevorzugt für eine ganze Zahl von 0 bis 3, steht, 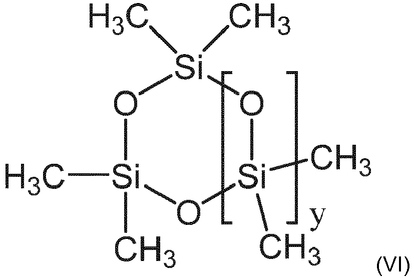 wobei y für eine ganze Zahl von 1 bis 5, bevorzugt für eine ganze Zahl von 1 bis 3, steht.

In einer weiteren bevorzugten Ausführungsform ist ein erfindungsgemäßes Verfahren gekennzeichnet,
dass das Mittel (a) mindestens ein Oligoalkylsiloxan enthält, das ausgewählt ist aus der Gruppe aus Hexamethyldisiloxan, Octamethyltrisiloxan, Decamethyltetrasiloxan, Hexamethylcyclotrisiloxan, Octamethylcyclotetrasiloxan und/oder Decamethylcyclopentasiloxan.

Im Gegensatz zu den reaktiven organischen Siliciumverbindungen, d.h. den Silanen der Formeln (I), (II) und (IV), sind die Oligoalkylsiloxane ausschließlich aus Dialkylsilyl-Gruppen (insbesondere Dimethylsilylgruppen) und Trialkylsilylgruppen (insbesondere Trimethylsilylgruppen) aufgebaut, die über Sauerstoffatome miteinander verknüpft sind. Damit sind die Oligoalkylsiloxane selbst keine reaktiven Verbindungen und besitzen auch keine hydrolysierbaren Gruppen.

Es konnte herausgefunden werden, dass die Mittel (a), die neben den organischen Siliciumverbindungen (Silanen) zusätzlich auch Oligoalkylsiloxane enthielten, eine besonders gute Lagerstabilität besaßen. Ohne auf diese Theorie beschränkt zu sein, wird vermutet, dass die reaktiven organischen Siliicumverbindungen (insbesondere die Silane Formeln (I), (II) und (IV)) sich sehr gut in den Oligoalkylsiloxane lösen, wobei der inerte Charakter der Oligoalkylsiloxane ein zu schnelles Abreagieren der Silane verhindert und die Silane vor Luftfeuchtigkeit schützt. Auf diese Weise reagieren die im Mittel (a) enthaltenen Silane nicht in unerwünschter Weise vorzeitig ab, und ihre Reaktivität blieb erhalten.

Bei Anwendung im Färbeverfahren zeigte sich, dass auf diese Weise unabhängig von den bei der Ausfärbung herrschenden Bedingungen ein konstant gutes Farbergebnis erzielt werden konnte. Ausfärbungen, die bei hoher Luftfeuchtigkeit vorgenommen wurden, waren genauso gut wie die bei niedrigerer Luftfeuchtigkeit applizierten Färbemittel.

Als Konsequenz hiervon konnte auch bei Einsatz der Mittel (a) im erfindungsgemäßen Verfahren eine besonders hohe Farbintensität erzielt werden, da sich bei Anwendung des Mittels (a) auf dem Keratinmaterial ein besonders homogener Film ausbildete, der noch genug reaktive Stellen besaß, um die direktziehenden Farbstoffe des Mittels (b) zu binden.

Dieser Effekt konnte insbesondere dann erreicht werden, wenn das Mittel (a) das oder die Oligoalkylsiloxane in bestimmten Mengenbereichen enthielt. Als ganz besonders bevorzugt hat es sich herausgestellt, wenn das Mittel (a) - bezogen auf das Gesamtgewicht des Mittels (a) - ein oder mehrere Oligoalkylsiloxane in einer Gesamtmenge von 0,1 bis 25,0 Gew.-%, bevorzugt 1,0 bis 15,0 Gew.-5, weiter bevorzugt 1,5 bis 10,0 Gew.-% und ganz besonders bevorzugt 2,0 bis 9,0 Gew.-% enthält.

In einer weiteren bevorzugten Ausführungsform ist ein erfindungsgemäßes Verfahren dadurch gekennzeichnet, dass das Mittel (a) - bezogen auf das Gesamtgewicht des Mittels (a) - ein oder mehrere Oligoalkylsiloxane in einer Gesamtmenge von 0,1 bis 25,0 Gew.-%, bevorzugt 1,0 bis 15,0 Gew.-5, weiter bevorzugt 1,5 bis 10,0 Gew.-% und ganz besonders bevorzugt 2,0 bis 9,0 Gew.-% enthält.

Zur optimalen Steuerung der Polymerisierungsgeschwindigkeit der Silane hat es sich weiterhin als besonders vorteilhaft herausgestellt, die organischen Siliciumverbindungen und die Oligoalkylsilxoane in bestimmten Mengenverhältnissen im Mittel (a) einzusetzen.

In einer weiteren bevorzugten Ausführungsform ist ein erfindungsgemäßes Verfahren dadurch gekennzeichnet, dass das Gewichtsverhältnis aus allen im Mittel (a) enthaltenen organischen Siliciumverbindungen der Formeln (I), (II) und (IV) zu allen im Mittel (a) enthaltenen Oligoalkylsiloxanen der Formel (V) und (VI) bei einem Wert von 5:1 bis 1:1, bevorzugt von 3:1 bis 1:1 und besonders bevorzugt von 2:1 bis 1:1 liegt.

Beispiel: Ein Mittel (a) enthält:
- 2,0 Gew.-% 3-Aminopropyltriethoxysilan und
- 7,0 Gew.-% Methyltrimethoxsilan und
- 5,0 Gew.-% Hexamethyldisiloxan

Das Gewichtsverhältnis aus allen im Mittel (a) enthaltenen organischen Siliciumverbindungen der Formeln (I), (II) und (IV) zu allen im Mittel (a) enthaltenen Oligoalkylsiloxanen der Formel (V) und (VI) liegt bei (2,0 + 7,0) / 5,0 = 1,8

Besonders resistente Färbungen konnten bei Anwendung eines alkalisch eingestellten Mittels (a) erhalten werden. Bevorzugt enthält das Mittel (a) Wasser und besitzt einen pH-Wert von 7,0 bis 11,5, bevorzugt von 7,5 bis 11,0 und besonders bevorzugt von 8,0 bis 10,5.

In einer weiteren ganz besonders bevorzugten Ausführungsform ist ein erfindungsgemäßes Verfahren dadurch gekennzeichnet, dass das Mittel (a) einen pH-Wert von 7,0 bis 11,5, bevorzugt von 7,5 bis 11,0 und besonders bevorzugt von 8,0 bis 10,5 besitzt.

### Mittel (b)

Das Mittel (b) ist gekennzeichnet durch seinen Gehalt an mindestens einem Pigment und/oder einem direktziehenden Farbstoff. Das Mittel (b) kann auch als Färbemittel (b) bezeichnet werden.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist ein erfindungsgemäßes Mittel (b) dadurch gekennzeichnet, dass es mindestens eine farbgebende Verbindung aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (Cl 77499), gelbes Eisenoxid (Cl 77492), rotes und braunes Eisenoxid (Cl 77491), Manganviolett (Cl 77742), Ultramarine (Natrium-Aluminiumsulfosilikate, Cl 77007, Pigment Blue 29), Chromoxidhydrat (Cl77289), Eisenblau (Ferric Ferrocyanide, Cl77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Verfahren dadurch gekennzeichnet, dass das Mittel (b) mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (b) mindestens eine farbgebende Verbindung aus der Gruppe der Pigmente enthält, die ausgewählt ist aus Pigmenten auf Mica- oder Glimmerbasis, die mit einem oder mehreren Metalloxiden aus der Gruppe aus Titandioxid (Cl 77891), schwarzem Eisenoxid (Cl 77499), gelbem Eisenoxid (Cl 77492), rotem und/oder braunem Eisenoxid (Cl 77491, Cl 77499), Manganviolett (Cl 77742), Ultramarine (Natrium-Aluminiumsulfosilikate, Cl 77007, Pigment Blue 29), Chromoxidhydrat (Cl 77289), Chromoxid (Cl 77288) und/oder Eisenblau (Ferric Ferrocyanide, Cl 77510) beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, Cl 77491 (IRON OXIDES)
Colorona Passion Orange, Merck, Mica, Cl 77491 (Iron Oxides), Alumina
Colorona Patina Silver, Merck, MICA, Cl 77499 (IRON OXIDES), Cl 77891 (TITANIUM DIOXIDE)
Colorona RY, Merck, Cl 77891 (TITANIUM DIOXIDE), MICA, Cl 75470 (CARMINE)
Colorona Oriental Beige, Merck, MICA, Cl 77891 (TITANIUM DIOXIDE), Cl 77491 (IRON OXIDES)
Colorona Dark Blue, Merck, MICA, TITANIUM DIOXIDE, FERRIC FERROCYANIDE
Colorona Chameleon, Merck, Cl 77491 (IRON OXIDES), MICA
Colorona Aborigine Amber, Merck, MICA, Cl 77499 (IRON OXIDES), Cl 77891 (TITANIUM DIOXIDE)
Colorona Blackstar Blue, Merck, Cl 77499 (IRON OXIDES), MICA
Colorona Patagonian Purple, Merck, MICA, Cl 77491 (IRON OXIDES), Cl 77891 (TITANIUM DIOXIDE), Cl 77510 (FERRIC FERROCYANIDE)
Colorona Red Brown, Merck, MICA, Cl 77491 (IRON OXIDES), Cl 77891 (TITANIUM DIOXIDE)
Colorona Russet, Merck, Cl 77491 (TITANIUM DIOXIDE), MICA, Cl 77891 (IRON OXIDES)
Colorona Imperial Red, Merck, MICA, TITANIUM DIOXIDE (Cl77891), D&C RED NO. 30 (Cl 73360)
Colorona Majestic Green, Merck, Cl 77891 (TITANIUM DIOXIDE), MICA, Cl 77288 (CHROMIUM OXIDE GREENS)
Colorona Light Blue, Merck, MICA, TITANIUM DIOXIDE (Cl 77891), FERRIC FERROCYANIDE (Cl 77510)
Colorona Red Gold, Merck, MICA, Cl 77891 (TITANIUM DIOXIDE), Cl 77491 (IRON OXIDES)
Colorona Gold Plus MP 25, Merck, MICA, TITANIUM DIOXIDE (Cl 77891), IRON OXIDES (Cl 77491)
Colorona Carmine Red, Merck, MICA, TITANIUM DIOXIDE, CARMINE
Colorona Blackstar Green, Merck, MICA, Cl 77499 (IRON OXIDES)
Colorona Bordeaux, Merck, MICA, Cl 77491 (IRON OXIDES)
Colorona Bronze, Merck, MICA, Cl 77491 (IRON OXIDES)
Colorona Bronze Fine, Merck, MICA, Cl 77491 (IRON OXIDES)
Colorona Fine Gold MP 20, Merck, MICA, Cl 77891 (TITANIUM DIOXIDE), Cl 77491 (IRON OXIDES)
Colorona Sienna Fine, Merck, Cl 77491 (IRON OXIDES), MICA
Colorona Sienna, Merck, MICA, Cl 77491 (IRON OXIDES)
Colorona Precious Gold, Merck, Mica, Cl 77891 (Titanium dioxide), Silica, Cl 77491(Iron oxides), Tin oxide
Colorona Sun Gold Sparkle MP 29, Merck, MICA, TITANIUM DIOXIDE, IRON OXIDES, MICA, Cl 77891, Cl 77491 (EU)
Colorona Mica Black, Merck, Cl 77499 (Iron oxides), Mica, Cl 77891 (Titanium dioxide)
Colorona Bright Gold, Merck, Mica, Cl 77891 (Titanium dioxide), Cl 77491 (Iron oxides)
Colorona Blackstar Gold, Merck, MICA, Cl 77499 (IRON OXIDES)
Weiterhin besonders bevorzugte Farbpigmente mit der Handelsbezeichnung Xirona^{®} sind beispielsweise:
   Xirona Golden Sky, Merck, Silica, Cl 77891 (Titanium Dioxide), Tin Oxide
   Xirona Caribbean Blue, Merck, Mica, Cl 77891 (Titanium Dioxide), Silica, Tin Oxide
   Xirona Kiwi Rose, Merck, Silica, Cl 77891 (Titanium Dioxide), Tin Oxide
   Xirona Magic Mauve, Merck, Silica, Cl 77891 (Titanium Dioxide), Tin Oxide.

Zudem sind besonders bevorzugte Farbpigmente mit der Handelsbezeichnung Unipure^{®} beispielsweise:
Unipure Red LC 381 EM, Sensient Cl 77491 (Iron Oxides), Silica
Unipure Black LC 989 EM, Sensient, Cl 77499 (Iron Oxides), Silica
Unipure Yellow LC 182 EM, Sensient, Cl 77492 (Iron Oxides), Silica

Im Rahmen einer weiteren Ausführungsform kann das erfindungsgemäße Mittel auch (b) ein oder mehrere farbgebende Verbindungen aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern Cl 42090, Cl 69800, Cl 69825, Cl 73000, Cl 74100, Cl 74160, gelbe Pigmente mit den Color Index Nummern Cl 11680, Cl 11710, Cl 15985, Cl 19140, Cl 20040, Cl 21100, Cl 21108, Cl 47000, Cl 47005, grüne Pigmente mit den Color Index Nummern Cl 61565, Cl 61570, Cl 74260, orange Pigmente mit den Color Index Nummern Cl 11725, Cl 15510, Cl 45370, Cl 71105, rote Pigmente mit den Color Index Nummern Cl 12085, Cl 12120, Cl 12370, Cl 12420, Cl 12490, Cl 14700, Cl 15525, Cl 15580, Cl 15620, Cl 15630, Cl 15800, Cl 15850, Cl 15865, Cl 15880, Cl 17200, Cl 26100, Cl 45380, Cl 45410, Cl 58000, Cl 73360, Cl 73915 und/oder Cl 75470 genannt werden.

In einer weiteren besonders bevorzugten Ausführungsform ist ein erfindungsgemäßes Verfahren dadurch gekennzeichnet, dass das Mittel (b) mindestens eine farbgebende Verbindungen aus der Gruppe der organischen Pigmente enthält, die ausgewählt ist aus der Gruppe aus Carmin, Chinacridon, Phthalocyanin, Sorgho, blaue Pigmente mit den Color Index Nummern Cl 42090, Cl 69800, Cl 69825, Cl 73000, Cl 74100, Cl 74160, gelbe Pigmente mit den Color Index Nummern Cl 11680, Cl 11710, Cl 15985, Cl 19140, Cl 20040, Cl 21100, Cl 21108, Cl 47000, Cl 47005, grüne Pigmente mit den Color Index Nummern CI 61565, CI 61570, CI 74260, orange Pigmente mit den Color Index Nummern CI 11725, Cl 15510, CI 45370, CI 71105, rote Pigmente mit den Color Index Nummern CI 12085, CI 12120, CI 12370, CI 12420, CI 12490, CI 14700, CI 15525, Cl 15580, CI 15620, CI 15630, CI 15800, CI 15850, CI 15865, CI 15880, CI 17200, CI 26100, CI 45380, Cl 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Mittel (b) des erfindungsgemäßen Verfahrens ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Das oder die Pigmente können in einer Menge von 0,001 bis 20 Gew.-%, insbesondere von 0,05 bis 5 Gew.-%, jeweils bezogen auf das Gesamtgewicht des Mittels (b), eingesetzt werden.

Als farbgebende Verbindungen können die im erfindungsgemäßen Verfahren eingesetzten Mittel (b) auch einen oder mehrere direktziehende Farbstoffe enthalten. Bei direktziehende Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L.

Direktziehende Farbstoffe können in anionische, kationische und nichtionische direktziehende Farbstoffe unterteilt werden.

In einer weiteren bevorzugten Ausführungsform ist ein erfindungsgemäßes Mittel dadurch gekennzeichnet, dass es als farbgebende Verbindung (b) mindestens einen anionischen, kationischen und/oder nichtionischen direktziehenden Farbstoff enthält.

In einer weiteren bevorzugten Ausführungsform ist ein erfindungsgemäßes Mittel dadurch gekennzeichnet, dass es (b) mindestens einen anionischen, kationischen und/oder nichtionischen direktziehenden Farbstoff enthält.

Geeignete kationische direktziehende Farbstoffe sind beispielsweise Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76

Als nichtionische direktziehende Farbstoffe können beipsielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl )-amino-2-nitrobenzol, 3-Nitro-4-(2-hydroxyethyl )-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

In einer weiteren bevorzugten Ausführungsform ist ein erfindungsgemäßes Verfahren dadruch gekennzeichnet, dass das Mittel (b) mindestens einen direktziehenden Farbstoff enthält, der ausgewählt ist aus der Gruppe der anionischen, der kationischen und der nichtionischen direktziehenden Farbstoffe.

Im Zuge der zu dieser Erfindung führenden Arbeiten hat sich herausgestellt, dass insbesondere mit Mitteln (b), die mindestens einen anionischen direktziehenden Farbstoff enthalten, Färbungen mit besonders hoher Farbintensität erzeugt werden können.

In einer explizit ganz besonders bevorzugten Ausführungsform ist ein im erfindungsgemäßes Verfahren eingesetztes Mittel (b) daher dadurch gekennzeichnet, dass es mindestens einen anionischen direktziehenden Farbstoff enthält.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO-, -SO₃⁻ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Im Rahmen einer Ausführungsform des Verfahrens bevorzugt ist damit der Einsatz eines Mittels (b) welches dadurch gekennzeichnet ist, dass es mindestens einen anionischen direktziehenden Farbstoff enthält, der ausgewählt ist aus der Gruppe der Nitrophenylendiamine, der Nitroaminophenole, der Azofarbstoffe, der Anthrachinonfarbstoffe, der Triarylmethanfarbstoffe, der Xanthen-Farbstoffe, der Rhodamin-Farbstoffe, der Oxazinfarbstoffen und/oder der Indophenolfarbstoffe, wobei die Farbstoffe aus der vorgenannten Gruppe jeweils mindestens eine Carbonsäuregruppe (-COOH), eine Natriumcarboxylatgruppe (-COONa), eine Kaliumcarboxylatgruppe (-COOK), eine Sulfonsäuregruppe (-SO₃H) eine Natriumsulfonatgruppe (-SO₃Na) und/oder eine Kaliumsulfonatgruppe (-SO₃K) besitzen.

Als besonders gut geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,Cl 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, Cl 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, Iodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der direktziehenden Farbstoffe kann beispielsweise auf dem folgenden Weg bestimmt werden. 0,1 g des anionischen Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%. Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

Ein ganz besonders bevorzugtes erfindungsgemäßes Verfahren ist dadurch gekennzeichnet, dass das Mittel (b) mindestens einen anionischen direktziehenden Farbstoff aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1 enthält.

Der oder die direktziehenden Farbstoffe, insbesondere die anionischen direktziehenden Farbstoffe, können je nach erwünschter Farbintensität in verschiedenen Mengen im Mittel (b) eingesetzt werden. Besonders gute Ergebnisse konnten erhalten werden, wenn das Mittel (b) - bezogen auf das Gesamtgewicht des Mittels (b) - einen oder mehrere direktziehende Farbstoffe in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthält.

In einer weiteren bevorzugten Ausführungsform ist ein Mittel (b) dadurch gekennzeichnet, dass es - bezogen auf das Gesamtgewicht des Mittels (b) - einen oder mehrere direktziehende Farbstoffe in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthält.

In einer weiteren bevorzugten Ausführungsform ist ein erfindungsgemäßes Mittel dadurch gekennzeichnet, dass es - bezogen auf das Gesamtgewicht des Mittels (b) - einen oder mehrere anionische direktziehende Farbstoffe (b) in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthält.

### Weitere Inhaltsstoffe in den Mitteln (a) und (b)

Die zuvor beschriebenen Mittel (a) und (b) können ferner auch noch ein oder mehrere optionale Inhaltsstoffe enthalten.

Die Mittel können zusätzlich ein oder mehrere Tenside enthalten. Unter dem Begriff Tenside werden grenzflächenaktive Substanzen verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂ - C₁₈ - Acylsarcosin.

Die Mittel können auch zusätzlich mindestens ein nichtionisches Tensid enthalten. Geeignete nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit guten Eigenschaften werden ebenfalls erhalten, wenn sie als nichtionische Tenside Fettsäureester von ethoxyliertem Glycerin enthalten, die mit mindestens 2 Mol Ethylenoxid umgesetzt wurden. Die nichtionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die Mittel zusätzlich auch mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind
- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die erfindungsgemäßen Mittel auch mindestens ein anionisches Tensid enthalten. Als anionische Tenside werden oberflächenaktive Mittel mit ausschließlich anionischen Ladungen (neutralisiert durch ein entsprechendes Gegenkation) bezeichnet. Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

Die anionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Zur Einstellung des gewünschten pH-Wertes können die Mittel (a), (b) und (c) auch mindestens ein Alkalisierungsmittel und/oder Acidifizierungsmittel enthalten. Bei den pH-Werten im Sinne der vorliegenden Erfindung handelt es sich um pH-Werte, die bei einer Temperatur von 22°C gemessen wurden.

Als Alkalisierungsmittel können die Mittel (a), (b) und (c) beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Dem Fachmann geläufige Acidifizierungsmittel sind beipsielsewiese Genuss-Säuren, wie beispielsweise Zitronensäure, Essigsäure, Äpfelsäure oder Weinsäure, sowie verdünnte Mineralsäuren, wie beispielsweise Salzsäure, Schwefelsäure oder Phosphorsäure.

Die Mittel können ferner auch noch weitere Wirk-, Hilfs- und Zusatzstoffe enthalten, wie beispielsweise Lösungsmittel, Fettbestandteile wie beispielsweise der C₈-C₃₀-Fettalkohole, der C₈-C₃₀-Fettsäuretriglyceride, der C₈-C₃₀-Fettsäuremonoglyceride, der C₈-C₃₀-Fettsäurediglyceride und/oder der Kohlenwasserstoffe; Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, N₂O, Dimethylether, CO₂ und Luft.

Ganz besonders bevorzugt enthält das Mittel (b) zusätzlich mindestens ein anionisches Polymer aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Verfahren zum Färben von Keratinmaterialien

Im Rahmen des erfindungsgemäßen Verfahrens werden die Mittel (a) und (b) auf die keratinischen Materialien, insbesondere auf die menschlichen Haare, appliziert. Damit sind die Mittel (a) und (b) die anwendungsbereiten Mittel. Die Mittel (a) und (b) sind voneinander verschieden.

Die Mittel (a) und (b) können prinzipiell gleichzeitig oder sukzessive angewendet werden, wobei die sukzessive Anwendung bevorzugt ist.

Die besten Ergebnisse konnten erhalten werden, wenn das Mittel (a) als Vorbehandlungsmittel auf die Keratinmaterialien gegeben wurde und danach das Mittel (b) als Färbemittel angewendet wurde.

Ganz besonders bevorzugt ist daher ein Verfahren zum Färben von keratinischem Material, insbesondere menschlichen Haaren, umfassend die folgenden Schritte in der angegebenen Reihenfolge:
- in einem ersten Schritt Anwendung eines Mittels (a) auf dem keratinischem Material, wobei das Mittel (a) mindestens eine organische Siliciumverbindung enthält, die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst, und wobei das Mittel (a) weiterhin mindestens ein Oligoalkylsiloxan enthält, und
- in einem zweiten Schritt Anwendung eines Mittels (b) auf dem keratinischen Material, wobei das Mittel (b) mindestens eine farbgebende Verbindung aus der Gruppe der Pigmente und/oder der direktziehenden Farbstoffe enthält.

Die Mittel (a),und (b) werden besonders bevorzugt innerhalb ein und desselben Färbeverfahrens angewendet, was bedeutet, dass zwischen der Anwendung der Mittel (a) und (b) ein Zeitraum von maximal einigen Stunden liegt.

Im Rahmen einer weiteren bevorzugten Ausführungsform ist ein erfindungsgemäßes Verfahren dadurch gekennzeichnet, das zunächst das Mittel (a) angewendet wird, und danach das Mittel (b) angewendet wird, wobei der Zeitraum zwischen der Anwendung der Mittel (a) und (b) bei maximal 24 Stunden, bevorzugt bei maximal 12 Stunden und besonders bevorzugt bei maximal 6 Stunden liegt.

Im Rahmen des erfindungsgemäßen Verfahrens werden die Keratinmaterialien, insbesondere die menschlichen Haare, zunächst mit Mittel (a) behandelt. Im Anschluss daran wird das eigentliche Färbemittel (b) - welches die farbgebenden Verbindungen enthält - auf die Keratinmaterialien gegeben.

Bevorzugt enthält das Mittel (a) selbst keine Farbstoffe bzw. keine farbgebenden Verbindungen. Kennzeichnend für das Vorbehandlungsmittel (a) ist sein Gehalt an mindestens einer reaktiven organische Siliciumverbindung und mindestens einem Oligoalkylsiloxan. Das oder die reaktiven organischen Siliciumverbindungen (a) funktionalisieren die Haaroberfläche, sobald sie mit dieser in Kontakt kommen. Auf diesem Wege wird ein erster, noch ungefärbter Film ausgebildet. Die Schnelligkeit, in der dieser Film im Färbeprozess ausgebildet wird, kann durch Zusatz des Oligoalkylsiloxans zum Mittel (a) auf die optimale Geschwindigkeit eingestellt werden. Im zweiten Schritt des Verfahrens wird nun ein Färbemittel (b) auf die Haare aufgetragen. Während der Anwendung des Färbemittels (b) gehen die farbgebenden Verbindungen eine Wechselwirkung mit dem Silan-Film ein und werden auf diese Weise an die Keratinmaterialien gebunden. Hierbei können die anwendungstechnischen Eigenschaften der resultierenden Färbung durch Wahl der optimalen Verfahrensbedingungen noch weiter verbessert werden.

Im Rahmen einerweiteren Ausführungsform ganz besonders bevorzugt ist ein Verfahren, umfassend die folgenden Schritte in der angegebenen Reihenfolge
(1) Anwendung des Mittels (a) auf dem keratinischen Material,
(2) Einwirken lassen des Mittels (a) für einen Zeitraum von 10 Sekunden bis 10 Minuten, bevorzugt von 10 Sekunden bis 5 Minuten,
(3) gegebenenfalls Ausspülen des keratinischen Materials mit Wasser,
(4) Anwendung des Mittels (b) auf dem keratinischen Material,
(5) Einwirken lassen des Mittels (b) für einen Zeitraum von 30 Sekunden bis 30 Minuten, bevorzugt von 30 Sekunden bis 10 Minuten, und
(6) Ausspülen des keratinischen Materials mit Wasser.

Unter dem Ausspülen des keratinischen Materials mit Wasser in den Schritten (3) und (6) des Verfahrens wird erfindungsgemäß verstanden, dass für den Ausspülvorgang nur Wasser verwendet wird, ohne dass noch weitere, von den Mitteln (a) und (b) verschiedene Mittel zur Anwendung kämen.

In einem Schritt (1) wird zunächst das Mittel (a) auf die Keratinmaterialien, insbesondere die menschlichen Haare, appliziert.

Nach dem Auftragen wird das Mittel (a) auf die Keratinmaterialien einwirken gelassen. In diesem Zusammenhang haben sich Einwirkzeiten von 10 Sekunden bis 10 Minuten, bevorzugt von 20 Sekunden bis 5 Minuten und ganz besonders bevorzugt von 30 Sekunden bis 2 Minuten auf den Haaren haben sich als besonders vorteilhaft erwiesen.

Im Rahmen einer bevorzugten Ausführungsform des erfindungsgemäßen Verfahrens kann das Mittel (a) nun von den Keratinmaterialien ausgespült werden, bevor das Mittel (b) im nachfolgenden Schritt auf die Haare appliziert wird.

Färbungen mit ebenfalls guten Waschechtheiten wurden erhalten, wenn das Mittel (b) auf die Keratinmaterialien appliziert wurde, die noch mit dem Mittel (a) beaufschlagt waren.

In Schritt (4) wird nun das Mittel (b) auf die Keratinmaterialien appliziert. Nach dem Auftragen wird nun das Mittel (b) auf die Haare einwirken gelassen.

Das erfindungsgemäße Verfahren erlaubt selbst bei kurzer Einwirkzeit des Mittels (b) die Erzeugung von Färbungen mit besonders guter Intensität und Waschechtheit. Einwirkzeiten von 10 Sekunden bis 10 Minuten, bevorzugt von 20 Sekunden bis 5 Minuten und ganz besonders bevorzugt von 30 Sekunden bis 3 Minuten auf den Haaren haben sich als besonders vorteilhaft erwiesen.

In Schritt (6) wird nun das Mittel (b) (sowie ggf. noch vorhandenes Mittel (a)) mit Wasser aus dem Keratinmaterial ausgespült.

Im Rahmen einerweiteren Ausführungsform ganz besonders bevorzugt ist ein Verfahren, umfassend die folgenden Schritte in der angegebenen Reihenfolge
(1) Anwendung des Mittels (a) auf dem keratinischen Material,
(2) Einwirken lassen des Mittels (a) für einen Zeitraum von 10 Sekunden bis 10 Minuten, bevorzugt von 10 Sekunden bis 5 Minuten,
(3) gegebenenfalls Ausspülen des keratinischen Materials mit Wasser,
(4) Anwendung des Mittels (b) auf dem keratinischen Material,
(5) Einwirken lassen des Mittels (b) für einen Zeitraum von 30 Sekunden bis 30 Minuten, bevorzugt von 30 Sekunden bis 10 Minuten, und
(6) Ausspülen des keratinischen Materials mit Wasser.

In Rahmen dieser Ausführungsform erfolgt die Abfolge der Schritte (1) bis (6) bevorzugt innerhalb von 24 Stunden.

### Mehrkomponenten-Verpackungseinheit (Kit-of-parts)

Im Rahmen des erfindungsgemäßen Verfahrens werden die Mittel (a) und (b) auf den Keratinmaterialien angewendet, d.h. bei den beiden Mitteln (a) und (b) handelt es sich jeweils um die anwendungsbereiten Mittel.

Zur Erhöhung des Anwenderkomforts werden dem Anwender bevorzugt alle benötigten Mittel in Form einer Mehrkomponenten-Verpackungseinheit (Kit-of-parts) zur Verfügung gestellt.

Ein zweiter Gegenstand der vorliegenden Erfindung ist daher eine Mehrkomponenten-Verpackungseinheit (Kit-of-parts) zum Färben von keratinischem Material, umfassend getrennt voneinander konfektioniert
- einen ersten Container mit einem Mittel (a), wobei das Mittel (a) mindestens eine organische Siliciumverbindung enthält, die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst, und wobei das Mittel (a) weiterhin mindestens ein Oligoalkylsiloxan enthält, und
- einen zweiten Container mit einem Mittel (b), wobei das Mittel (b) mindestens eine farbgebende Verbindung aus der Gruppe der Pigmente und/oder der direktziehenden Farbstoffe enthält.

Die im Mittel (a) des Kits enthaltenen organische Siliciumverbindungen entsprechen den organischen Siliciumverbindungen, die auch im Mittel (a) des zuvor beschriebenen Verfahrens eingesetzt wurden.

Die im Mittel (a) des Kits enthaltenen Oligoalkylsiloxane entsprechen den Oligoalkylsiloxane, die auch im Mittel (a) des zuvor beschriebenen Verfahrens eingesetzt wurden.

Die im Mittel (b) des Kits enthaltenen farbgebenden Verbindungen aus der Gruppe der Pigmente und/oder der direktziehenden Farbstoffe entsprechen den farbgebenden Verbindungen aus der Gruppe der Pigmente und/oder der direktziehenden Farbstoffe, die auch im Mittel (b) des zuvor beschriebenen Verfahrens eingesetzt wurden.

Ein zweiter Gegenstand der vorliegenden Erfindung ist daher eine Mehrkomponenten-Verpackungseinheit (Kit-of-parts) zum Färben von keratinischem Material, umfassend getrennt voneinander konfektioniert
- einen ersten Container mit einem Mittel (a), wobei das Mittel (a) mindestens eine organische Siliciumverbindung enthält, die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst, und wobei das Mittel (a) weiterhin mindestens ein Oligoalkylsiloxan enthält, wobei organische Siliciumverbindung und Oligoalkylsiloxan bei Beschreibung des ersten Erfindungsgegenstands im Detail offenbart wurden
- einen zweiten Container mit einem Mittel (b), wobei das Mittel (b) mindestens eine farbgebende Verbindung aus der Gruppe der Pigmente und/oder der direktziehenden Farbstoffe enthält, wie sie bei Beschreibung des ersten Erfindungsgegenstands im Detail offenbart wurde.

Das Mittel (a) enthält mit der oder den organischen Siliciumverbindungen eine Klasse von hochreaktiven Verbindungen, die wie zuvor beschriebenen in Anwesenheit von Wasser eine Hydrolyse bzw. Oligomerisierung und/oder Polymerisieurng eingehen können. Infolge ihrer hohen Reaktivität bilden diese organischen Siliciumverbindungen auf dem Keratinmaterial einen Film aus. Zur Verhinderung einer vorzeitigen Oligomerisierung bzw. Polymerisierung und zur Steuerung der Polymerisierungsgeschwindigkiet enthält das Mittel (a) zusätzlich mindestens ein Oligoalkylsiloxan.

Zur Vermeidung der vorzeitigen Oligomerisierung bzw. Polymerisierung ist es für den Anwender von wesentlichem Vorteil, das anwendungsbereite Mittel (a) erst kurz vor der Anwendung herstellen.

Im Rahmen einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (a1), wobei das Mittel (a1) mindestens eine organische Siliciumverbindung enthält, die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst, und wobei das Mittel (a1) weiterhin mindestens ein Oligoalkylsiloxan enthält, und
- einen zweiten Container mit einem Mittel (a2), wobei das Mittel (2) Wasser enthält, und
- einen dritten Container mit einem Mittel (b), wobei das Mittel (b) mindestens eine farbgebende Verbindung aus der Gruppe der Pigmente und/oder der direktziehenden Farbstoffe enthält.

Um eine möglichst lagerstabile Formulierung bereitstellen zu können, wird das Mittel (a1) selbst bevorzugt wasserarm oder wasserfrei konfektioniert.

In einer bevorzugten Ausführungsform ist eine erfindungsgemäße Mehrkomponenten-Verpackungseinheit (Kit-of-parts) dadurch gekennzeichnet, dass das Mittel (a1) - bezogen auf das Gesamtgewicht des Mittels (a1) - einen Wassergehalt von 0,001 bis 10,0 Gew.-%, bevorzugt von 0,5 bis 9,0 Gew.-%, weiter bevorzugt von 1,0 bis 8,0 Gew.-% und ganz besonders bevorzugt von 1,5 bis 7,0 Gew.-% enthält.

Das Mittel (a2) enthält Wasser. In einer bevorzugten Ausführungsform ist eine erfindungsgemäße Mehrkomponenten-Verpackungseinheit (Kit-of-parts) dadurch gekennzeichnet, dass das Mittel (a2) - bezogen auf das Gesamtgewicht des Mittels (a2) - einen Wassergehalt von 15 bis 100 Gew.-%, bevorzugt von 35 bis 100 Gew.-%, weiter bevorzugt von 55 bis 100 Gew.-%, noch weiter bevorzugt von 65 bis 100 Gew.-% und ganz besonders bevorzugt von 75 bis 100 Gew.-% besitzt.

Innerhalb dieser Ausführungsform wird das anwendungsbereite Mittel (a) nun durch Vermischen der Mittel (a1) und (a2) hergestellt.

Beispielsweise kann der Anwender das Mittel (a1), welches die organische Siliciumverbindung(en) enthält, zunächst mit dem wasserhaltigen Mittel (a2) verrühren oder verschütteln. Diese Mischung aus (a1) und (a2) kann der Anwender nun - entweder direkt nach ihrer Herstellung oder nach einer kurzen Reaktionszeit von 10 Sekunden bis 20 Minuten - auf die Keratinmaterialien applizieren. Im Anschluss daran kann der Anwender wie zuvor beschrieben das Mittel (b) anwenden.

In einer bevorzugten Ausführungsform ist eine erfindungsgemäße Mehrkomponenten-Verpackungseinheit (Kit-of-parts) dadurch gekennzeichnet, dass das Mittel (a1) - bezogen auf das Gesamtgewicht des Mittels (a1) - eine oder mehrere organische Siliciumverbindungen der Formel (I) und/oder (II) in einer Gesamtmenge von 1,0 bis 35,0 Gew.-%, bevorzugt 3,0 bis 25,0 Gew.-%, weiter bevorzugt 6,0 bis 20,0 Gew.-% und ganz besonders bevorzugt 9,0 bis 15,0 Gew.-% enthält.

In einer bevorzugten Ausführungsform ist eine erfindungsgemäße Mehrkomponenten-Verpackungseinheit (Kit-of-parts) dadurch gekennzeichnet, dass das Mittel (a1) - bezogen auf das Gesamtgewicht des Mittels (a1) - eine oder mehrere organische Siliciumverbindungen der Formel (IV) in einer Gesamtmenge von 1,0 bis 80,0 Gew-%, bevorzugt 10,0 bis 70,0 Gew.-%, weiter bevorzugt von 20,0 bis 60,0 Gew.-% und ganz besonders bevorzugt von 30,0 bis 60,0 Gew.-% enthält.

In einer bevorzugten Ausführungsform ist eine erfindungsgemäße Mehrkomponenten-Verpackungseinheit (Kit-of-parts) dadurch gekennzeichnet, dass das Mittel (a1) - bezogen auf das Gesamtgewicht des Mittels (a1) - eine oder mehrere Oligoalkylsiloxane in einer Gesamtmenge von 5,0 bis 50,0 Gew.-% bevorzugt von 10,0 bis 45,0 Gew.-%, weiter bevorzugt von 15,0 bis 40,0 Gew.-% und ganz besonders bevorzugt von 20,0 bis 35,0 Gew.-% enthält.

Betreffend die weiteren bevorzugten Ausführungsformen der erfindungsgemäßen Mehrkomponenten-Verpackungseinheit gilt *mutatis mutantis* das zum erfindungsgemäßen Verfahren gesagte.

Beispiele

### 1. Formulierungen

Es wurden die folgenden Formulierungen hergestellt:

### Vorbehandlungsmittel (a)

| Mittel (a1) | (a1V1) Vergleich | (a1V2) Vergleich | (a1E) Erfindung |
|---|---|---|---|
| (3-Aminopropyl)triethoxysilan | 13,3 g | 13,3 g | 13,3 g |
| Methyltrimethoxysilan | 46,7 g | 46,7 g | 46,7 g |
| Hexamethyldisiloxan | --- | --- | 33,3 g |
| Wasser | 40,0 g | --- | 6,7 g |

| Mittel (a2) | (a2) |
|---|---|
| Ammoniak/Zitronensäure | ad pH 9,5 |
| Wasser | 100 g |

### Färbemittel (b)

| | (b) |
|---|---|
| Acid Violet 43 | 2,0 g |
| PVP K 30 (Ashland, ISP, Polyvinylpyrrolidone) | 4,5 g |
| Dermacryl 79 (Akzo Nobel, Acrylates/Octylacrylamide Copolymer, CAS-Nr. 129702-02-9) | 4,5 g |
| Ammoniak (25 %ige wässrige Lösung) | ad pH 10 |
| Wasser | ad 100 g |

### 2. Lagertest

Jeweils 5 g des Mittels (a1V1), (a1V2) und (a1E) wurden direkt nach der Herstellung in eine Glasflasche gefüllt. Jede Glasflasche wurde für 1 Stunde offen stehen gelassen. Danach wurde jede Glasflasche verschlossen und für 10 Wochen bei 25 °C gelagert.

| | (a1V1) Vergleich | (a1V2) Vergleich | (a1E) Erfindung |
|---|---|---|---|
| 10 Wochen, 25 °C | schlierig, Gallerte | schlierig, leicht gelartig | klare Lösung |

### 3. Anwendung

Zur Herstellung des anwendungsbereiten Vorbehandlungsmittels (a) wurden die jeweils angegebene Menge des gelagerten Mittels (a1V1), (a1V2) bzw. (a1E) mit der angegebenen Menge des Mittels (a2) unter Schütteln vermischt. Danach wurde das Mittel (a) für 15 Minuten stehen gelassen. Das Mittel (a) ist das anwendungsbereite Mittel.

Jeweils eine Haarsträhne (Kerling, Euronaturhaar weiß) wurde in das Mittel (a) getaucht und für 1 Minuten darin belassen. Danach wurde überflüssiges Mittel (a) von jeder Haarsträhne gestreift. Jede Haarsträhne wurde mit Wasser ausgewaschen. Überschüssiges Wasser wurde von jeder Haarsträhne gestreift.

Im Anschluss daran wurden die Haarsträhnen jeweils in das Mittel (b) getaucht und für 1 Minute darin belassen. Danach wurde überflüssiges Mittel (b) von jeder Haarsträhne gestreift. Jede Haarsträhne wurde getrocknet und visuell bewertet. Danach wurde jede Haarsträhne gründlich (1 Minute) mit Wasser ausgewaschen, getrocknet und erneut visuell bewertet.

| Bsp | 1 | 2 | 3 |
|---|---|---|---|
| Mittel (a1), gelagert 10 Wochen, 25 °C | (a1V1) 8,4 g | (a1V2) 5g | (a1E) 8,4 g |
| Mittel (a2) | 91,6 g | 95 g | 91,6 g |

| Mittel (b) | (b) | (b) | (b) |
|---|---|---|---|
| Färbung vor dem Auswaschen | schwach violett + | violett ++ | violett +++ |
| Färbung nach dem Auswaschen | nahezu ungefärbt - | schwach violett + | violett +++ |

| | | | |
|---|---|---|---|
| Farbintensität: - = ungefärbt + = gering ++ = mittel +++ = sehr gut | | | |

## Patentansprüche

1. Verfahren zum Färben von keratinischem Material, insbesondere menschlichen Haaren, umfassend die folgenden Schritte:
- Anwendung eines Mittels (a) auf dem keratinischem Material, wobei das Mittel (a) mindestens eine organische Siliciumverbindung enthält, die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst, und wobei das Mittel (a) weiterhin mindestens ein Oligoalkylsiloxan enthält, und
- Anwendung eines Mittels (b) auf dem keratinischen Material, wobei das Mittel (b) mindestens eine farbgebende Verbindung aus der Gruppe der Pigmente und/oder der direktziehenden Farbstoffe enthält.

2. Verfahren nach Anspruch 1, **dadurch gekennzeichnet, dass** das Mittel (a) mindestens eine organische Siliciumverbindung der Formel (I) und/oder (II) enthält
R₁R₂N-L-Si(OR₃)ₐ(R₄)_{b} (I),
wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃, R₄ unabhängig voneinander für eine C₁-C₆-Alkylgruppe stehen,
- a, für eine ganze Zahl von 1 bis 3 steht, und
- b für die ganze Zahl 3 - a steht, und
wobei in der organischen Siliciumverbindung der Formel (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R5, R5', R5", R6, R6' und R6" unabhängig voneinander für eine C₁-C₆-Alkylgruppe stehen,
- A, A', A", A‴ und Aʺʺ unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (III) stehen
- (Aʺʺ)-Si(R₆")_{d}"(OR₅")_{c}" (III),
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
- mit der Maßgabe, dass mindestens einer der Reste aus e, f, g und h von 0 verschieden ist.

3. Verfahren nach einem der Ansprüche 1 bis 2, **dadurch gekennzeichnet, dass** das Mittel (a) mindestens eine organische Siliciumverbindung der Formel (I) enthält,
R₁R₂N-L-Si(OR₃)ₐ(R₄)_{b} (I),
wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen und
- a für die Zahl 3 steht und
- b für die Zahl 0 steht.

4. Verfahren nach einem der Ansprüche 1 bis 3, **dadurch gekennzeichnet, dass** das Mittel (a) mindestens eine organische Siliciumverbindung der Formel (I) enthält, die ausgewählt ist aus der Gruppe aus
- (3-Aminopropyl)trimethoxysilan
- (3-Aminopropyl)triethoxysilan
- (2-Aminoethyl)trimethoxysilan
- (2-Aminoethyl)triethoxysilan
- (3-Dimethylaminopropyl)trimethoxysilan
- (3-Dimethylaminopropyl)triethoxysilan
- (2-Dimethylaminoethyl)trimethoxysilan und/oder
- (2-Dimethylaminoethyl)triethoxysilan.

5. Mittel nach einem der Ansprüche 1 bis 4, **dadurch gekennzeichnet, dass** das Mittel (a) mindestens eine organische Siliciumverbindung der Formel (II) enthält,
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
wobei
- e und f beide für die Zahl 1 stehen,
- g und h beide für die Zahl 0 stehen,
- A und A' unabhängig voneinander für eine lineare, zweiwertige C₁-C₆-Alkylengruppe stehen und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

6. Mittel nach einem der Ansprüche 1 bis 5, **dadurch gekennzeichnet, dass** es (a) mindestens eine organische Siliciumverbindung der Formel (II) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und/oder
- N,N-Bis[3-(triethoxysilyl)propyl]-2-Propen-1-amin.

7. Verfahren nach einem der Ansprüche 1 bis 6, **dadurch gekennzeichnet, dass** das Mittel (a) mindestens eine organische Siliciumverbindung der Formel (IV) enthält.
R₉Si(OR₁₀)ₖ(R₁₁)ₘ (IV),
wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

8. Verfahren nach einem der Ansprüche 1 bis 7, **dadurch gekennzeichnet, dass** das Mittel (a) mindestens eine organische Siliciumverbindung der Formel (IV) enthält, die ausgewählt ist aus der Gruppe aus
- Methyltrimethoxysilan
- Methyltriethoxysilan
- Ethyltrimethoxysilan
- Ethyltriethoxysilan
- Octyltrimethoxysilan
- Octyltriethoxysilan
- Dodecyltrimethoxysilan und/oder
- Dodecyltriethoxysilan.

9. Verfahren nach einem der Ansprüche 1 bis 8, **dadurch gekennzeichnet, dass** das Mittel (a) - bezogen auf das Gesamtgewicht des Mittels (a) - eine oder mehrere organische Siliciumverbindungen in einer Gesamtmenge von 0,1 bis 20,0 Gew.-%, bevorzugt 1,0 bis 15,0 Gew.-% und besonders bevorzugt 2,0 bis 8,0 Gew.-% enthält.

10. Verfahren nach einem der Ansprüche 1 bis 9, **dadurch gekennzeichnet, dass** das Mittel (a) - bezogen auf das Gesamtgewicht des Mittels (a) - eine oder mehrere organische Siliciumverbindungen der Formel (I) und/oder (II) in einer Gesamtmenge von 0,1 bis 10,0 Gew.-%, bevorzugt 0,5 bis 5,0 Gew.-% und besonders bevorzugt 1,0 bis 3,0 Gew.-% enthält.

11. Verfahren nach einem der Ansprüche 1 bis 10, **dadurch gekennzeichnet, dass** das Mittel (a) - bezogen auf das Gesamtgewicht des Mittels (a) - eine oder mehrere organische Siliciumverbindungen der Formel (IV) in einer Gesamtmenge von 0,1 bis 20,0 Gew.-%, bevorzugt 2,0 bis 15,0 Gew.-% und besonders bevorzugt 4,0 bis 9,0 Gew.-% enthält.

12. Verfahren nach einem der Ansprüche 1 bis 11, **dadurch gekennzeichnet, dass** das Mittel (a) mindestens ein lineares und/oder cyclisches Oligoalkylsiloxan enthält.

13. Verfahren nach einem der Ansprüche 1 bis 12, **dadurch gekennzeichnet, dass** das Mittel (a) mindestens ein Oligoalkylsiloxan der Formel (V) und/oder (VI) enthält, 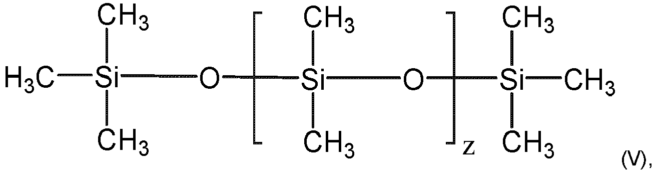 wobei z für eine ganze Zahl von 0 bis 10, bevorzugt für eine ganze Zahl von 0 bis 3, steht, 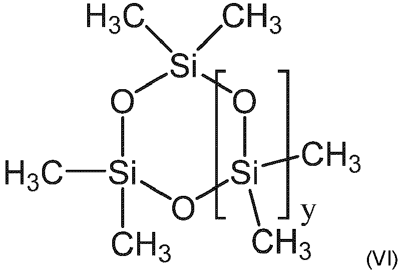 wobei y für eine ganze Zahl von 1 bis 5, bevorzugt für eine ganze Zahl von 1 bis 3, steht.

14. Verfahren nach einem der Ansprüche 1 bis 13, **dadurch gekennzeichnet, dass** das Mittel (a) mindestens ein Oligoalkylsiloxan enthält, das ausgewählt ist aus der Gruppe aus Hexamethyldisiloxan, Octamethyltrisiloxan, Decamethyltetrasiloxan, Hexamethylcyclotrisiloxan, Octamethylcyclotetrasiloxan und Decamethylcyclopentasiloxan.

15. Verfahren nach einem der Ansprüche 1 bis 14, **dadurch gekennzeichnet, dass** das Mittel (a) - bezogen auf das Gesamtgewicht des Mittels (a) - ein oder mehrere Oligoalkylsiloxane in einer Gesamtmenge von 0,1 bis 25,0 Gew.-%, bevorzugt 1,0 bis 15,0 Gew.-5, weiter bevorzugt 1,5 bis 10,0 Gew.-% und ganz besonders bevorzugt 2,0 bis 9,0 Gew.-% enthält.

16. Verfahren nach einem der Ansprüche 1 bis 15, **dadurch gekennzeichnet, dass** das Gewichtsverhältnis aus allen im Mittel (a) enthaltenen organischen Siliciumverbindungen der Formeln (I), (II) und (IV) zu allen im Mittel (a) enthaltenen Oligoalkylsiloxanen der Formel (V) und (VI) bei einem Wert von 5:1 bis 1:1, bevorzugt von 3:1 bis 1:1 und besonders bevorzugt von 2:1 bis 1:1 liegt.

17. Verfahren nach einem der Ansprüche 1 bis 16, **dadurch gekennzeichnet, dass** das Mittel (b) mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

18. Verfahren nach einem der Ansprüche 1 bis 17, **dadurch gekennzeichnet, dass** das Mittel (b) mindestens eine farbgebende Verbindungen aus der Gruppe der organischen Pigmente enthält, die bevorzugt ausgewählt ist aus der Gruppe aus Carmin, Chinacridon, Phthalocyanin, Sorgho, blaue Pigmente mit den Color Index Nummern CI 42090, CI 69800, Cl 69825, CI 73000, Cl 74100, Cl 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, Cl 45410, Cl 58000, CI 73360, CI 73915 und/oder CI 75470.

19. Verfahren nach einem der Ansprüche 1 bis 18, **dadurch gekennzeichnet, dass** das Mittel (b) mindestens einen direktziehenden Farbstoff enthält, der bevorzugt ausgewählt ist aus der Gruppe der anionischen, der kationischen und der nichtionischen direktziehenden Farbstoffe.

20. Verfahren nach einem der Ansprüche 1 bis 19, **dadurch gekennzeichnet, dass** das Mittel (b) mindestens einen anionischen direktziehenden Farbstoff aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1 enthält.

21. Verfahren nach einem der Ansprüche 1 bis 20, **dadurch gekennzeichnet**, das zunächst das Mittel (a) angewendet wird und danach das Mittel (b) angewendet wird, wobei der Zeitraum zwischen der Anwendung der Mittel (a) und (b) bei maximal 24 Stunden, bevorzugt bei maximal 12 Stunden und besonders bevorzugt bei maximal 6 Stunden liegt.

22. Verfahren nach einem der Ansprüche 1 bis 21, umfassend die folgenden Schritte in der angegebenen Reihenfolge
(1) Anwendung des Mittels (a) auf dem keratinischen Material,
(2) Einwirken lassen des Mittels (a) für einen Zeitraum von 10 Sekunden bis 10 Minuten, bevorzugt von 10 Sekunden bis 5 Minuten,
(3) gegebenenfalls Ausspülen des keratinischen Materials mit Wasser,
(4) Anwendung des Mittels (b) auf dem keratinischen Material,
(5) Einwirken lassen des Mittels (b) für einen Zeitraum von 30 Sekunden bis 30 Minuten, bevorzugt von 30 Sekunden bis 10 Minuten, und
(6) Ausspülen des keratinischen Materials mit Wasser.

23. Mehrkomponenten-Verpackungseinheit (Kit-of-parts) zum Färben von keratinischem Material, umfassend getrennt voneinander konfektioniert
- einen ersten Container mit einem Mittel (a), wobei das Mittel (a) mindestens eine organische Siliciumverbindung enthält, die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst, und wobei das Mittel (a) weiterhin mindestens ein Oligoalkylsiloxan enthält, wie sie in den Ansprüchen 1 bis 16 beschrieben sind, und
- einen zweiten Container mit einem Mittel (b), wobei das Mittel (b) mindestens eine farbgebende Verbindung aus der Gruppe der Pigmente und/oder der direktziehenden Farbstoffe enthält, wie sie in den Ansprüchen 1, 17, 18, 19 und/oder 20 beschrieben sind.

24. Mehrkomponenten-Verpackungseinheit (Kit-of-parts) nach Anspruch 23, umfassend getrennt voneinander konfektioniert
- einen ersten Container mit einem Mittel (a1), wobei das Mittel (a1) mindestens eine organische Siliciumverbindung enthält, die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst, und wobei das Mittel (a1) weiterhin mindestens ein Oligoalkylsiloxan enthält, wie sie in den Ansprüchen 1, 17, 18, 19 und/oder 20 beschrieben sind,
und
- einen zweiten Container mit einem Mittel (a2), wobei das Mittel (2) Wasser enthält und
- einen dritten Container (b), wobei das Mittel (b) mindestens eine farbgebende Verbindung aus der Gruppe der Pigmente und/oder der direktziehenden Farbstoffe enthält, wie sie in den Ansprüchen 1, 17, 18, 19 und/oder 20 beschrieben sind.

25. Mehrkomponenten-Verpackungseinheit (Kit-of-parts) nach Anspruch 24, **dadurch gekennzeichnet, dass** das Mittel (a1) - bezogen auf das Gesamtgewicht des Mittels (a1) - einen Wassergehalt von 0,001 bis 10,0 Gew.-%, bevorzugt von 0,5 bis 9,0 Gew.-%, weiter bevorzugt von 1,0 bis 8,0 Gew.-% und ganz besonders bevorzugt von 1,5 bis 7,0 Gew.-% besitzt.

26. Mehrkomponenten-Verpackungseinheit (Kit-of-parts) nach einem der Ansprüche 24 bis 25, **dadurch gekennzeichnet, dass** das Mittel (a2) - bezogen auf das Gesamtgewicht des Mittels (a2) - einen Wassergehalt von 15 bis 100 Gew.-%, bevorzugt von 35 bis 100 Gew.-%, weiter bevorzugt von 55 bis 100 Gew.-%, noch weiter bevorzugt von 65 bis 100 Gew.-% und ganz besonders bevorzugt von 75 bis 100 Gew.-% besitzt.

## Claims

1. A method for dyeing keratin material, in particular human hair, comprising the following steps:
- applying an agent (a) to the keratin material, wherein agent (a) contains at least one organic silicon compound selected from silanes having one, two or three silicon atoms, wherein the organic silicon compound comprises one or more hydroxyl groups and/or hydrolyzable groups per molecule, and wherein agent (a) further contains at least one oligoalkylsiloxane, and
- applying an agent (b) to the keratin material, wherein agent (b) contains at least one coloring compound from the group of pigments and/or substantive dyes.

2. The method according to claim 1, **characterized in that** agent (a) contains at least one organic silicon compound of formula (I) and/or (II),
R₁R₂N-L-Si(OR₃)ₐ(R₄)_{b} (I),
where
- R₁ and R₂ represent, independently of one another, a hydrogen atom or a C₁-C₆ alkyl group,
- L represents a linear or branched, divalent C₁-C₂₀ alkylene group,
- R₃ and R₄ represent, independently of one another, a C₁-C₆ alkyl group,
- a represents an integer from 1 to 3, and
- b represents the integer 3 - a,
where, in the organic silicon compound of formula (II),
(R₅O)_{c}(R₆)_{d}Si-(A)ₑ-[NR₇-(A')_{f}-[O-(A")]_{g}-[NR₈-(A‴)]ₕ-Si(R₆)_{d'}(OR₅')_{c'} (II),
- R5, R5', R5", R6, R6' and R6" represent, independently of one another, a C₁-C₆ alkyl group,
- A, A', A", A'" and Aʺʺ represent, independently of one another, a linear or branched, divalent C₁-C₂₀ alkylene group, and
- R₇ and R₈ represent, independently of one another, a hydrogen atom, a C₁-C₆ alkyl group, a hydroxy C₁-C₆ alkyl group, a C₂-C₆ alkenyl group, an amino C₁-C₆ alkyl group or a group of formula (III),
- (Aʺ)-Si(R₆")_{d}"(OR₅")_{c}" (III),
- c represents an integer from 1 to 3,
- d represents the integer 3 - c,
- c' represents an integer from 1 to 3,
- d' represents the integer 3 - c',
- c" represents an integer from 1 to 3,
- d" represents the integer 3 - c",
- e represents 0 or 1,
- f represents 0 or 1,
- g represents 0 or 1, and
- h represents 0 or 1,
- with the proviso that at least one of e, f, g and h is different from 0.

3. The method according to claim 1 or 2, **characterized in that** agent (a) contains at least one organic silicon compound of formula (I),
R₁R₂N-L-Si(OR₃)ₐ(R₄)_{b} (I),
where
- R₁ and R₂ both represent a hydrogen atom,
- L represents a linear, divalent C₁-C₆ alkylene group, preferably a propylene group (-CH₂-CH₂-CH₂-) or an ethylene group (-CH₂-CH₂-),
- R₃ and R₄ represent, independently of one another, a methyl group or an ethyl group,
- a represents the number 3, and
- b represents the number 0.

4. The method according to any of claims 1 to 3, **characterized in that** agent (a) contains at least one organic silicon compound of formula (I) selected from the group consisting of:
- (3-aminopropyl)trimethoxysilane,
- (3-aminopropyl)triethoxysilane,
- (2-aminoethyl)trimethoxysilane,
- (2-aminoethyl)triethoxysilane,
- (3-dimethylaminopropyl)trimethoxysilane,
- (3-dimethylaminopropyl)triethoxysilane,
- (2-dimethylaminoethyl)trimethoxysilane, and/or
- (2-dimethylaminoethyl)triethoxysilane.

5. An agent according to one of claims 1 to 4, **characterized in that** agent (a) contains at least one organic silicon compound of formula (II),
(R₅O)_{c}(R₆)_{d}Si-(A)ₑ-[NR₇₋(A')]_{f}-[O-(A")]_{g}-[NR₈-(A‴)]ₕ-Si(R₆)_{d'}(OR₅')_{c'} (II),
where
- e and f both represent the number 1,
- g and h both represent the number 0,
- A and A' represent, independently of one another, a linear, divalent C₁-C₆ alkylene group, and
- R7 represents a hydrogen atom, a methyl group, a 2-hydroxyethyl group, a 2-alkenyl group, a 2-aminoethyl group or a group of formula (III).

6. The agent according to one of claims 1 to 5, **characterized in that** it contains (a) at least one organic silicon compound of formula (II) selected from the group consisting of:
- 3-(trimethoxysilyl)-N-[3-(trimethoxysilyl)propyl]-1-propanamine,
- 3-(triethoxysilyl)-N-[3-(triethoxysilyl)propyl]-1-propanamine,
- N-methyl-3-(trimethoxysilyl)-N-[3-(trimethoxysilyl)propyl]-1-propanamine,
- N-methyl-3-(triethoxysilyl)-N-[3-(triethoxysilyl)propyl]-1-propanamine,
- 2-[bis[3-(trimethoxysilyl)propyl]amino]ethanol,
- 2-[bis[3-(triethoxysilyl)propyl]amino]ethanol,
- 3-(trimethoxysilyl)-N,N-bis[3-(trimethoxysilyl)propyl]-1-propanamine,
- 3-(triethoxysilyl)-N,N-bis[3-(triethoxysilyl)propyl]-1-propanamine,
- N1,N1-bis[3-(trimethoxysilyl)propyl]-1,2-ethanediamine,
- N1,N1-bis[3-(triethoxysilyl)propyl]-1,2-ethanediamine,
- N,N-bis[3-(trimethoxysilyl)propyl]-2-propen-1-amine, and/or
- N,N-bis[3-(triethoxysilyl)propyl]-2-propen-1-amine.

7. A method according to one of claims 1 to 6, **characterized in that** agent (a) contains at least one organic silicon compound of formula (IV),
R₉Si(OR₁₀)ₖ(R₁₁)ₘ (IV),
where
- R₉ represents a C₁-C₁₂ alkyl group,
- R¹⁰ represents a hydrogen atom or a C₁-C₆ alkyl group,
- R₁₁ represents a C₁-C₆ alkyl group,
- k represents an integer from 1 to 3, and
- m is the integer 3 - k.

8. The method according to one of claims 1 to 7, **characterized in that** agent (a) contains at least one organic silicon compound of formula (IV) selected from the group consisting of:
- methyltrimethoxysilane,
- methyltriethoxysilane,
- ethyltrimethoxysilane,
- ethyltriethoxysilane,
- octyltrimethoxysilane,
- octyltriethoxysilane,
- dodecyltrimethoxysilane, and/or
- dodecyltriethoxysilane.

9. The method according to one of claims 1 to 8, **characterized in that** agent (a) contains one or more organic silicon compounds in a total amount of from 0.1 to 20.0 wt.%, preferably 1.0 to 15.0 wt.%, and particularly preferably 2.0 to 8.0 wt.%, based on the total weight of agent (a).

10. The method according to one of claims 1 to 9, **characterized in that** agent (a) contains one or more organic silicon compounds of formula (I) and/or (II) in a total amount of from 0.1 to 10.0 wt.%, preferably 0.5 to 5.0 wt.%, and particularly preferably 1.0 to 3.0 wt.%, based on the total weight of agent (a).

11. The method according to one of claims 1 to 10, **characterized in that** agent (a) contains one or more organic silicon compounds of formula (IV) in a total amount of from 0.1 to 20.0 wt.%, preferably 2.0 to 15.0 wt.%, and particularly preferably 4.0 to 9.0 wt.%, based on the total weight of agent (a).

12. The method according to one of claims 1 to 11, **characterized in that** agent (a) contains at least one linear and/or cyclic oligoalkylsiloxane.

13. The method according to one of claims 1 to 12, **characterized in that** agent (a) contains at least one oligoalkylsiloxane of formula (V) and/or (VI), 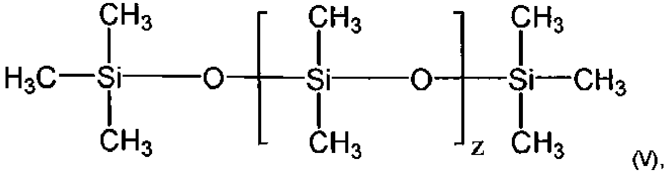 where z represents an integer from 0 to 10, preferably an integer from 0 to 3, and 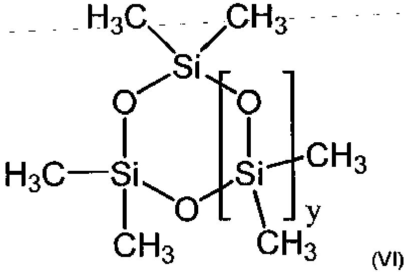 where y represents an integer from 1 to 5, preferably an integer from 1 to 3.

14. The method according to one of claims 1 to 13, **characterized in that** agent (a) contains at least one oligoalkylsiloxane selected from the group consisting of hexamethyldisiloxane, octamethyltrisiloxane, decamethyltetrasiloxane, hexamethylcyclotrisiloxane, octamethylcyclotetrasiloxane and decamethylcyclopentasiloxane.

15. The method according to one of claims 1 to 14, **characterized in that** agent (a) contains one or more oligoalkylsiloxanes in a total amount of from 0.1 to 25.0 wt.%, preferably 1.0 to 15.0 wt.%, more preferably 1.5 to 10.0 wt.%, and very particularly preferably 2.0 to 9.0 wt.%, based on the total weight of agent (a).

16. The method according to one of claims 1 to 15, **characterized in that** the weight ratio of all the organic silicon compounds of formulas (I), (II) and (IV) contained in agent (a) to all the oligoalkylsiloxanes of formulas (V) and (VI) contained in agent (a) is from 5:1 to 1:1, preferably from 3:1 to 1:1, and particularly preferably from 2:1 to 1:1.

17. The method according to one of claims 1 to 16, **characterized in that** agent (b) contains at least one coloring compound from the group of pigments selected from the group of colored metal oxides, metal hydroxides, metal oxide hydrates, silicates, metal sulfides, complex metal cyanides, metal sulphates, bronze pigments and/or from mica-based colored pigments which are coated with at least one metal oxide and/or a metal oxychloride.

18. The method according to one of claims 1 to 17, **characterized in that** agent (b) contains at least one coloring compound from the group of organic pigments, preferably selected from the group of carmine, quinacridone, phthalocyanine, sorghum, blue pigments with the Color Index numbers CI 42090, CI 69800, CI 69825, CI 73000, CI 74100 or CI 74160, yellow pigments with the Color Index numbers CI 11680, CI 11710, CI 15985, CI 19140, CI 20040, CI 21100, CI 21108, CI 47000 or CI 47005, green pigments with the Color Index numbers CI 61565, CI 61570 or CI 74260, orange pigments with the Color Index numbers CI 11725, CI 15510, CI 45370 or CI 71105, and red pigments with the Color Index numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 and/or CI 75470.

19. The method according to one of claims 1 to 18, **characterized in that** agent (b) contains at least one substantive dye which is preferably selected from the group consisting of anionic, cationic and non-ionic substantive dyes.

20. The method according to one of claims 1 to 19, **characterized in that** agent (b) contains at least one anionic substantive dye from the group consisting of Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 and/or D&C Brown 1.

21. The method according to one of claims 1 to 20, **characterized in that** agent (a) is applied first and then agent (b) is applied, the period between the application of agents (a) and (b) being at most 24 hours, preferably at most 12 hours, and particularly preferably at most 6 hours.

22. The method according to one of claims 1 to 21, comprising the following steps in the sequence indicated:
(1) applying agent (a) to the keratin material,
(2) allowing agent (a) to act for a period of 10 seconds to 10 minutes, preferably 10 seconds to 5 minutes,
(3) optionally rinsing the keratin material with water,
(4) applying agent (b) to the keratin material,
(5) allowing agent (b) to act for a period of 30 seconds to 30 minutes, preferably 30 seconds to 10 minutes, and
(6) rinsing the keratin material with water.

23. A multi-component packaging unit (kit-of-parts) for coloring keratin material, comprising, packaged separately from one another,
- a first container having an agent (a), wherein agent (a) contains at least one organic silicon compound selected from silanes having one, two or three silicon atoms, wherein the organic silicon compound comprises one or more hydroxyl groups and/or hydrolyzable groups per molecule, and wherein agent (a) further contains at least one oligoalkylsiloxane, as described in claims 1 to 16,
and
- a second container having an agent (b), wherein agent (b) contains at least one coloring compound from the group of pigments and/or substantive dyes, as described in claims 1, 17, 18, 19 and/or 20.

24. The multi-component packaging unit (kit-of-parts) according to claim 23, comprising, packaged separately from one another,
- a first container having an agent (a1), wherein agent (a1) contains at least one organic silicon compound selected from silanes having one, two or three silicon atoms, wherein the organic silicon compound comprises one or more hydroxyl groups and/or hydrolyzable groups per molecule, and wherein agent (a1) further contains at least one oligoalkylsiloxane, as described in claims 1, 17, 18, 19 and/or 20,
and
- a second container having an agent (a2), wherein agent (2) contains water, and
- a third container (b), wherein agent (b) contains at least one coloring compound from the group of pigments and/or substantive dyes, as described in claims 1, 17, 18, 19 and/or 20.

25. The multi-component packaging unit (kit-of-parts) according to claim 24, **characterized in that** agent (a1) has a water content of from 0.001 to 10.0 wt.%, preferably from 0.5 to 9.0 wt.%, more preferably from 1.0 to 8.0 wt.%, and very particularly preferably from 1.5 to 7.0 wt.%, based on the total weight of agent (a1).

26. The multi-component packaging unit (kit-of-parts) according to claim 24 or 25, **characterized in that** agent (a2) has a water content of from 15 to 100 wt.%, preferably from 35 to 100 wt.%, more preferably from 55 to 100 wt.%, even more preferably from 65 to 100 wt.%, and very particularly preferably from 75 to 100 wt.%, based on the total weight of agent (a2).

## Revendications

1. Procédé destiné à teindre de la matière kératinique, en particulier des cheveux humains, comprenant les étapes suivantes :
- application d'un agent (a) sur la matière kératinique, l'agent (a) contenant au moins un composé organique au silicium choisi parmi les silanes comportant un, deux ou trois atomes de silicium, le composé organique au silicium comprenant un ou plusieurs groupes hydroxyle et/ou groupes hydrolysables par molécule, et l'agent (a) contenant en outre au moins un oligoalkylsiloxane, et
- application d'un agent (b) sur la matière kératinique, l'agent (b) contenant au moins un composé colorant du groupe des pigments et/ou des colorants à action directe.

2. Procédé selon la revendication 1, **caractérisé en ce que** l'agent (a) contient au moins un composé organique au silicium de formule (I) et/ou (II)
R₁R₂N-L-Si(OR₃)ₐ(R₄)_{b} (I),
où
- R₁, R₂ représentent indépendamment l'un de l'autre un atome d'hydrogène ou un groupe alkyle en C₁-C₆,
- L représente un groupe alkylène divalent en C₁-C₂₀, linéaire ou ramifié,
- R₃, R₄ représentent indépendamment l'un de l'autre un groupe alkyle en C₁-C₆,
- a représente un nombre entier de 1 à 3, et
- b représente le nombre entier 3-a, et
où, dans le composé organique au silicium de formule (II)
(R₅O)_{c}(R₆)_{d}Si-(A)ₑ-[NR₇-(A')_{f}-[O-(A")]_{g}-[NR₈-(A‴)]ₕ-Si(R₆')_{d'}(OR₅')_{c'} (II),
- R5, R5', R5", R6, R6' et R6" représentent indépendamment les uns des autres un groupe alkyle en C₁-C₆,
- A, A', A", A'" et Aʺʺ représentent indépendamment les uns des autres un groupe alkylène divalent en C₁-C₂₀, linéaire ou ramifié,
- R₇ et R₈ représentent indépendamment l'un de l'autre un atome d'hydrogène, un groupe alkyle en C₁-C₆, un groupe hydroxy alkyle en C₁-C₆, un groupe alcényle en C₂-C₆, un groupe amino alkyle en C₁-C₆ ou un groupement de formule (III)
- (Aʺʺ)-Si(R₆")_{d}"(OR₅")_{c}" (III),
où
- c représente un nombre entier de 1 à 3,
- d représente le nombre entier 3-c,
- c' représente un nombre entier de 1 à 3,
- d' représente le nombre entier 3-c',
- c" représente un nombre entier de 1 à 3,
- d" représente le nombre entier 3-c",
- e représente 0 ou 1,
- f représente 0 ou 1,
- g représente 0 ou 1,
- h représente 0 ou 1,
- à condition qu'au moins l'un des radicaux parmi e, f, g et h soit différent de 0.

3. Procédé selon l'une des revendications 1 à 2, **caractérisé en ce que** l'agent (a) contient au moins un composé organique au silicium de formule (I),
R₁R₂N-L-Si(OR₃)ₐ(R₄)_{b} (I),
où
- R₁, R₂ représentent tous deux un atome d'hydrogène, et
- L représente un groupe alkylène divalent linéaire en C₁-C₆, de préférence un groupe propylène (-CH₂-CH₂-CH₂-) ou un groupe éthylène (-CH₂-CH₂-),
- R₃, R₄ représentent indépendamment l'un de l'autre un groupe méthyle ou un groupe éthyle, et
- a représente le nombre 3 et
- b représente le nombre 0.

4. Procédé selon l'une des revendications 1 à 3, **caractérisé en ce que** l'agent (a) contient au moins un composé organique au silicium de formule (I) qui est choisi dans le groupe constitué par
- (3-aminopropyl)triméthoxysilane
- (3-aminopropyl)triéthoxysilane
- (2-aminoéthyl)triméthoxysilane
- (2-aminoéthyl)triéthoxysilane
- (3-diméthylaminopropyl)triméthoxysilane
- (3-diméthylaminopropyl)triéthoxysilane
- (2-diméthylaminoéthyl)triméthoxysilane et/ou
- (2-diméthylaminoéthyl)triéthoxysilane.

5. Agent selon l'une des revendications 1 à 4, **caractérisé en ce que** l'agent (a) contient au moins un composé organique au silicium de formule (II),
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
où
- e et f représentent tous deux le nombre 1,
- g et h représentent tous deux le nombre 0,
- A et A' représentent indépendamment l'un de l'autre un groupe alkylène divalent linéaire en C₁-C₆ et
- R7 représente un atome d'hydrogène, un groupe méthyle, un groupe 2-hydroxyéthyle, un groupe 2-alcényle, un groupe 2-aminoéthyle ou un groupement de formule (III).

6. Agent selon l'une des revendications 1 à 5, **caractérisé en ce qu'**il (a) contient au moins un composé organique au silicium de formule (II) qui est choisi dans le groupe constitué par
- 3-(triméthoxysilyl)-N-[3-(triméthoxysilyl)propyl]-1-propanamine
- 3-(triéthoxysilyl)-N-[3-(triéthoxysilyl)propyl]-1-propanamine
- N-méthyl-3-(triméthoxysilyl)-N-[3-(triméthoxysilyl)propyl]-1-propanamine
- N-méthyl-3-(triéthoxysilyl)-N-[3-(triéthoxysilyl)propyl]-1-propanamine
- 2-[bis[3-(triméthoxysilyl)propyl]amino]-éthanol
- 2-[bis[3-(triéthoxysilyl)propyl]amino]-éthanol
- 3-(triméthoxysilyl)-N,N-bis[3-(triméthoxysilyl)propyl]-1-propanamine
- 3-(triéthoxysilyl)-N,N-bis[3-(triéthoxysilyl)propyl]-1-propanamine
- N1,N1-bis[3-(triméthoxysilyl)propyl]-1,2-éthanediamine,
- N1,N1-bis[3-(triéthoxysilyl)propyl]-1,2-éthanediamine,
- N,N-bis[3-(triméthoxysilyl)propyl]-2-propène-1-amine et/ou
- N,N-bis[3-(triéthoxysilyl)propyl]-2-propène-1-amine.

7. Procédé selon l'une des revendications 1 à 6, **caractérisé en ce que** l'agent (a) contient au moins un composé organique de silicium de formule (IV)
R₉Si(OR₁₀)ₖ(R₁₁)ₘ (IV),
où
- Rg représente un groupe alkyle en C₁-C₁₂,
- R₁₀ représente un atome d'hydrogène ou un groupe alkyle en C₁-C₆,
- R₁₁ représente un groupe alkyle en C₁-C₆,
- k représente un nombre entier de 1 à 3, et
- m représente le nombre entier 3-k.

8. Procédé selon l'une des revendications 1 à 7, **caractérisé en ce que** l'agent (a) contient au moins un composé organique au silicium de formule (IV) qui est choisi dans le groupe constitué par
- méthyltriméthoxysilane
- méthyltriéthoxysilane
- éthyltriméthoxysilane
- éthyltriéthoxysilane
- octyltriméthoxysilane
- octyltriéthoxysilane
- dodécyltriméthoxysilane et/ou
- dodécyltriéthoxysilane.

9. Procédé selon l'une des revendications 1 à 8, **caractérisé en ce que** l'agent (a) contient, par rapport au poids total de l'agent (a), un ou plusieurs composés organiques au silicium en une quantité totale de 0,1 à 20,0 % en poids, de préférence de 1,0 à 15,0 % en poids et de manière particulièrement préférée de 2,0 à 8,0 % en poids.

10. Procédé selon l'une des revendications 1 à 9, **caractérisé en ce que** l'agent (a) contient, par rapport au poids total de l'agent (a), un ou plusieurs composés organiques au silicium de formule (I) et/ou (II) en une quantité totale de 0,1 à 10,0 % en poids, de préférence de 0,5 à 5,0 % en poids et de manière particulièrement préférée de 1,0 à 3,0 % en poids.

11. Procédé selon l'une des revendications 1 à 10, **caractérisé en ce que** l'agent (a) contient, par rapport au poids total de l'agent (a), un ou plusieurs composés organiques au silicium de formule (IV) en une quantité totale de 0,1 à 20,0 % en poids, de préférence de 2,0 à 15,0 % en poids et de manière particulièrement préférée de 4,0 à 9,0 % en poids.

12. Procédé selon l'une des revendications 1 à 11, **caractérisé en ce que** l'agent (a) contient au moins un oligoalkylsiloxane linéaire et/ou cyclique.

13. Procédé selon l'une des revendications 1 à 12, **caractérisé en ce que** l'agent (a) contient au moins un oligoalkylsiloxane de formule (V) et/ou (VI), 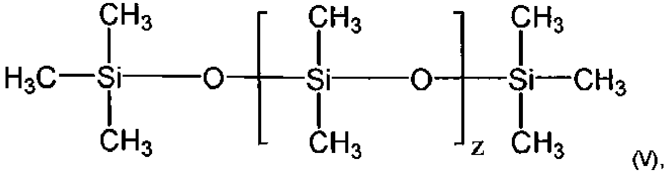 où z représente un nombre entier entre 0 et 10, de préférence un nombre entier entre 0 et 3, 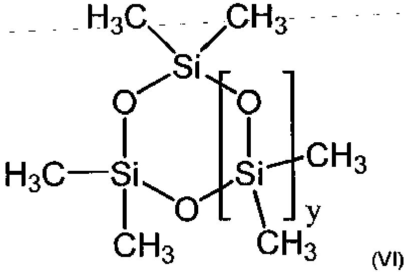 où y représente un nombre entier entre 1 et 5, de préférence un nombre entier entre 1 et 3.

14. Procédé selon l'une des revendications 1 à 13, **caractérisé en ce que** l'agent (a) contient au moins un oligoalkylsiloxane choisi dans le groupe constitué par l'hexaméthyldisiloxane, l'octaméthyltrisiloxane, le décaméthyltétrasiloxane, l'hexaméthylcyclotrisiloxane, l'octaméthylcyclotétrasiloxane et le décaméthylcyclopentasiloxane.

15. Procédé selon l'une des revendications 1 à 14, **caractérisé en ce que** l'agent (a) contient, par rapport au poids total de l'agent (a), un ou plusieurs oligoalkylsiloxanes en une quantité totale de 0,1 à 25,0 % en poids, de préférence de 1,0 à 15,0 % en poids, plus préférablement de 1,5 à 10,0 % en poids et de manière particulièrement préférée de 2,0 à 9,0 % en poids.

16. Procédé selon l'une des revendications 1 à 15, **caractérisé en ce que** le rapport pondéral de tous les composés organiques au silicium de formules (I), (II) et (IV) contenus dans l'agent (a) à l'ensemble des oligoalkylsiloxanes de formules (V) et (VI) contenus dans l'agent (a) est de 5:1 à 1:1, de préférence de 3:1 à 1:1 et de manière particulièrement préférée de 2:1 à 1:1.

17. Procédé selon l'une des revendications 1 à 16, **caractérisé en ce que** l'agent (b) contient au moins un composé colorant du groupe des pigments et qui est choisi dans le groupe constitué par les oxydes métalliques colorés, les hydroxydes métalliques, les oxydes métalliques hydratés, les silicates, les sulfures métalliques, les cyanures métalliques complexes, les sulfates métalliques, les pigments de bronze et/ou les pigments colorés à base de mica recouverts par au moins un oxyde métallique et/ou un oxychlorure métallique.

18. Procédé selon l'une des revendications 1 à 17, **caractérisé en ce que** l'agent (b) contient au moins un composé colorant du groupe des pigments organiques et choisi de préférence dans le groupe constitué par le carmin, la quinacridone, la phtalocyanine, le sorgho, les pigments bleus avec les numéros d'indice de couleur CI 42090, CI 69800, CI 69825, CI 73000, CI 74100, CI 74160, les pigments jaunes avec les numéros d'indice de couleur CI 11680, CI 11710, CI 15985, CI 19140, CI 20040, CI 21100, CI 21108, CI 47000, CI 47005, les pigments verts avec les numéros d'indice de couleur CI 61565, CI 61570, CI 74260, les pigments orange avec les numéros d'indice de couleur CI 11725, CI 15510, CI 45370, CI 71105, les pigments rouges avec les numéros d'indice de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

19. Procédé selon l'une des revendications 1 à 18, **caractérisé en ce que** l'agent (b) contient au moins un colorant à action directe qui est de préférence choisi dans le groupe constitué par les colorants à action directe anioniques, cationiques et non ioniques.

20. Procédé selon l'une des revendications 1 à 19, **caractérisé en ce que** l'agent (b) contient au moins un colorant à action directe anionique du groupe constitué par Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 et/ou D&C Brown 1.

21. Procédé selon l'une des revendications 1 à 20, **caractérisé en ce que** l'agent (a) est d'abord appliquém puis l'agent (b) est appliqué, la durée entre l'application des agents (a) et (b) étant au maximum de 24 heures, de préférence au maximum de 12 heures et de manière particulièrement préférée au maximum de 6 heures.

22. Procédé selon l'une des revendications 1 à 21, comprenant, dans l'ordre indiqué les étapes consistant à
(1) appliquer l'agent (a) sur la matière kératinique,
(2) laisser agir l'agent (a) pendant une durée de 10 secondes à 10 minutes, de préférence de 10 secondes à 5 minutes,
(3) rincer éventuellement la matière kératinique avec de l'eau,
(4) appliquer l'agent (b) sur la matière kératinique,
(5) laisser agir l'agent (b) pendant une durée de 30 secondes à 30 minutes, de préférence de 30 secondes à 10 minutes, et
(6) rincer la matière kératinique avec de l'eau.

23. Unité d'emballage à plusieurs composants (kit de pièces) destinée à teindre de la matière kératinique, comprenant, conditionnés séparés les uns des autres,
- un premier récipient comportant un agent (a), l'agent (a) contenant au moins un composé organique au silicium choisi parmi les silanes comportant un, deux ou trois atomes de silicium, le composé organique au silicium comprenant un ou plusieurs groupes hydroxyle et/ou groupes hydrolysables par molécule, et l'agent (a) contenant en outre au moins un oligoalkylsiloxane tel que décrit dans les revendications 1 à 16,
et
- un deuxième récipient contenant un agent (b), l'agent (b) contenant au moins un composé colorant du groupe des pigments et/ou colorants à action directe, tel que décrit dans les revendications 1, 17, 18, 19 et/ou 20.

24. Unité d'emballage à plusieurs composants (kit de pièces) selon la revendication 23, comprenant, conditionnés séparés les uns des autres,
- un premier récipient comportant un agent (a1), l'agent (a1) contenant au moins un composé organique au silicium choisi parmi les silanes comportant un, deux ou trois atomes de silicium, le composé organique au silicium comprenant un ou plusieurs groupes hydroxyle et/ou groupes hydrolysables par molécule, et l'agent (a1) contenant en outre au moins un oligoalkylsiloxane tel que décrit dans les revendications 1, 17, 18, 19 et/ou 20,
et
- un deuxième récipient comportant un agent (a2), l'agent (2) contenant de l'eau, et
- un troisième récipient (b), l'agent (b) contenant au moins un composé colorant du groupe des pigments et/ou colorants à action directe, tel que décrit dans les revendications 1, 17, 18, 19 et/ou 20.

25. Unité d'emballage à plusieurs composants (kit de pièces) selon la revendication 24, **caractérisée en ce que** l'agent (a1) contient, par rapport au poids total de l'agent (a1), une teneur en eau comprise entre 0,001 et 10,0 % en poids, de préférence entre 0,5 et 9,0 % en poids, plus préférablement entre 1,0 et 8,0 % en poids et de manière particulièrement préférée entre 1,5 et 7,0 % en poids.

26. Unité d'emballage à plusieurs composants (kit de pièces) selon l'une des revendications 24 à 25, **caractérisée en ce que** l'agent (a2) contient, par rapport au poids total de l'agent (a2), une teneur en eau de 15 à 100 % en poids, de préférence de 35 à 100 % en poids, plus préférablement de 55 à 100 % en poids, encore plus préférablement de 65 à 100 % en poids et de manière particulièrement préférée de 75 à 100 % en poids.
